(19) Europäisches Patentamt
European Patent Office
Office européen des brevets

(11) EP 4 574 212 A1

(12) **EUROPEAN PATENT APPLICATION**

(43) Date of publication:
**25.06.2025 Bulletin 2025/26**

(21) Application number: **23307357.6**

(22) Date of filing: **22.12.2023**

(51) International Patent Classification (IPC):
***A61P 31/00*** *(2006.01)* ***A61P 35/00*** *(2006.01)*
***C07C 259/06*** *(2006.01)* ***C07C 275/24*** *(2006.01)*
***C07C 323/44*** *(2006.01)* ***C07D 249/04*** *(2006.01)*
***C07D 413/12*** *(2006.01)* ***C07K 7/64*** *(2006.01)*

(52) Cooperative Patent Classification (CPC):
**C07K 7/64; A61P 31/00; A61P 35/00; C07C 259/06; C07C 275/24; C07C 323/44; C07D 249/04**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicants:
- **Centre National de la Recherche Scientifique 75016 Paris (FR)**
- **Université de Montpellier 34090 Montpellier (FR)**
- **Ecole Nationale Supérieure de Chimie de Montpellier 34296 Montpellier Cedex 5 (FR)**

(72) Inventors:
- **LOPEZ, Marie 34170 Castelnau-le-Lez (FR)**
- **GILBERT, Julie 34090 Montpellier (FR)**

(74) Representative: **LLR 2, rue Jean Lantier 75001 Paris (FR)**

Remarks:
The complete document including Reference Table(s) and the Sequence Listing(s) can be downloaded from the EPO website

(54) **HISTONE DEACETYLASE INHIBITORS**

(57) The invention relates to a compound of formula R-A-R, wherein R is a C4-C6 alcohol or an hydroxamic acid of formula (I)

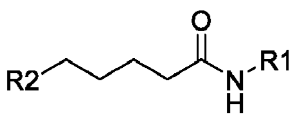
(I),

wherein
R1 is OH,
R2 is a C1-C2 alkyl, substituted by an amide group a triazol group , an amide group,
and wherein A is a C5-C11 alkyl, a group $(CH_2\text{-}CH_2O\text{-})_n$ wherein n is a integer from 1 to 40, α,ω-diamino- or α,ω-diazido-alkyl, a triazol or a compound comprising two or four amine or azide groups allowing the covalent link with both R residues



Processed by Luminess, 75001 PARIS (FR)

## Description

The Invention relates to histone deacetylase inhibitors.

WHO experts predict that the global population will reach 8 billion in 2020, the incidence of cancer will reach 20 million, and the death rate will reach 12 million. Cancer will become the first killer of human beings in the new century and pose the most serious threat to human survival.

In urban regions, cancer accounts first for the overall cause of death, while in rural regions, cancer accounts second for the overall cause of death. With the rapid increase in cancer morbidity and mortality.

HDAC inhibitors are widely used in a variety of cancers and can be combined with a variety of drugs to enhance its therapeutic effect, it is a well-recognized anti-tumor target. In the nucleus, histone deacetylase (HDAC) and histone acetyltransferase (HAT) jointly regulate gene transcription. In cancer cells, overexpression of HDAC leads to an increase in deacetylation, thereby increasing the attraction between DNA and histones, making the nucleosomes very tight, which is detrimental to the expression of tumor suppressor genes. By increasing histone acetylation, HDACI inhibitor (HDACi) can regulate the apoptosis and differentiate the expression of related proteins, and induce apoptosis and differentiation, therefore become a new class of anti-tumor drugs. Besides, HDAC is also involved in the regulation of many metabolic diseases, such as Alzheimer's disease, Parkinson's disease (Parkinson's disease) and other diseases, HDACi showed good results in animal and human experiments.

HDACi are therefore potent therapies for many diseases, but need to be improved.

Multivalency plays a large role in many biological and synthetic systems. The past 20 years of research have seen an explosion in the study of multivalent drug delivery systems based on scaffolds such as dendrimers, polymers, and other nanoparticles. Recent theoretical studies on multivalency indicate that the field may have had a misplaced emphasis on maximizing binding strength where in fact it is the selectivity of multivalent drug delivery systems that is the key to success.

One aim of the invention is to provide new HDACi that could enhance the efficacy of the known compounds, enhancing therefore the treatments of pathologies.

The invention relates to a compound of formula $(R)_2A(R')_2$,

wherein R and R' independently from each other are H, C4-C6 alcohol or an hydroxamic acid of formula (I)

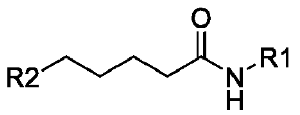
(I),

provided that R and R' are not simultaneously H,
wherein
R1 is OH, or
R2 is a C1-C12 alkyl group, possibly substituted by an amide group, a triazol group, an amide group, or an acid group, or a combination thereof,
and wherein A is a C5-C11 alkyl, a group $(CH_2\text{-}CH_2O\text{-})_n$ wherein n is an integer from 1 to 40, α,ω-diamino- or α,ω-diazido-aikyl, a triazol or a compound comprising two or four amine or azide groups, or any group allowing the covalent link with both R residues and/or both R' residues.

Advantageously, R' is H, and R is as defined above, *i.e*. a C4-C6 alcohol or an hydroxamic acid of formula (I) as defined above.

In other words, advantageously, the invention relates to a compound of formula $(R)_2A$, or R-A-R, wherein R is C4-C6 alcohol or an hydroxamic acid of formula (I)

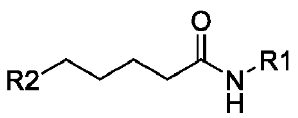
(I),

wherein

R1 is OH, or
R2 is a C1-C12 alkyl group, substituted by an amide group, a triazol group, an acid group, a phenyl group or an amide group, or a combination thereof,

and wherein A is a C5-C11 alkyl, a group $(CH_2-CH_2O-)_n$ wherein n is an integer from 1 to 40, α,ω-diamino- or α,ω-diazido-aikyl, a triazol or a compound comprising two amine or azide groups, or any group allowing the covalent link with both R residues.

The invention is based on the unexpected observation made by the inventors that multivalent inhibitors are significantly more efficient compared to the corresponding monovalent inhibitors. The compound according to the invention $(R)_2A(R')_2$ is constituted by a platform A liable to be grafted, by covalent bonds to 2 molecules of the same inhibitor (R), or 4 molecules of the same inhibitor (when R=R') or 2 molecules of a first inhibitor (R) and 2 molecules of a second inhibitor (R'). It is advantageous that the platform A is grafted to 2 molecules of the same inhibitor.

A is defined as containing 2 nitrogen atoms, onto which, *via* a covalent bound, R, or R' residues, are linked.

A contains thus 2 or 4 nitrogen atoms, in order to form either a R-A-R compound, when A contain solely 2 nitrogen atoms, or $(R)_2A(R')_2$ when A contain 4 nitrogen atoms.

Advantageously, the invention relates to the compound as defined above, A is chosen from the group consisting in a C5-C11 alkyl, a group $(CH_2-CH_2O-)_n$ wherein n is an integer from 1 to 40, α,ω-diamino- or α,ω-diazido-aikyl, a triazol, an aryl diamine, a C1-C3 alkyl diamine or diacide, a peptide, cyclic or not, comprising two reactive amino acids chosen from lysine, serine, aspartic acid, glutamic acid, cysteine, threonine, and tyrosine.

A is defined in the invention such that it contains reactive groups liable to form a covalent bound with R2 as defined above.

Advantageous A according to the invention is triazol, an aryl di- to tetraamine, or a peptide, cyclic or not, comprising two reactive amino acids chosen from lysine, serine, asparactic acid, glutamic acid, cysteine, threonine and tyrosine.

More advantageously, the invention relates to the compound as defined above, wherein the peptide is a 6-12-amino acids peptide, in particular a cyclic peptide, preferably a cyclic 10-amino acids peptide comprising 2 lysines, more preferably comprising solely 2 lysines. According to the invention, when A is a peptide, it is advantageously a cyclic peptide having 6, or 7, or 8, or 9, or 10, or 11, or 12 amino acids. This peptide contains or comprises 2 lysines, which means that it can contain 2 or 3 or 4 lysines. It is more advantageous that the peptide solely contains 2 lysines, and that the other amino acids be different from lysine. More advantageously, the peptide, does not contain other amino acid having a lateral chain comprising a nitrogen atom, i.e. does contain neither asparagine, histidine, nor tryptophane.

Advantageously, the invention relates to the compound as defined above, wherein R is chosen from:

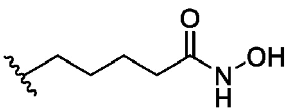 ; 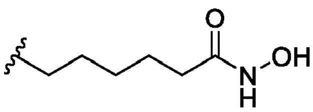 ; 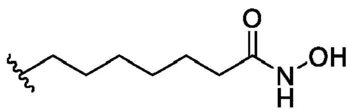

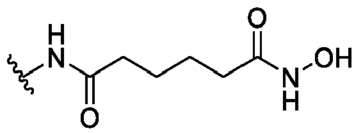 ; 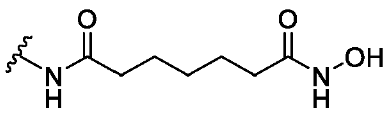 ; 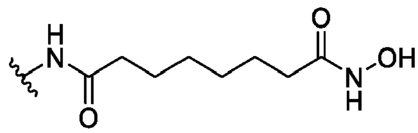

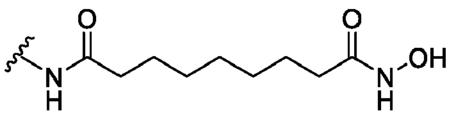 ; 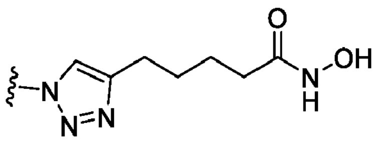

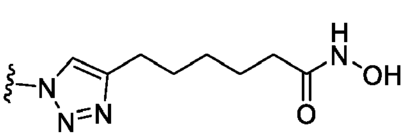 ; 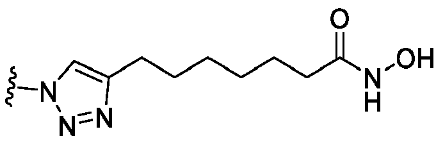 ;

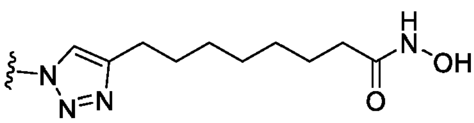 ; 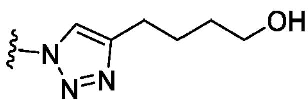 ;

and

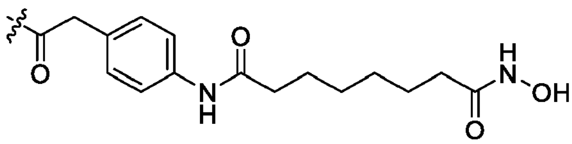

.

More advantageously, the invention relates to the compound as defined above, wherein A is chosen from:

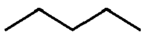 ; 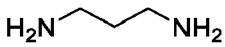 ; 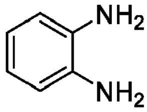 ; 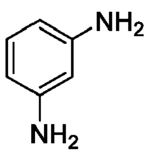 ; 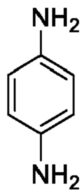 ; 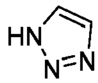

or a 10-amino acids peptide having a sequence as set force in the following sequence XX1 XXK/RXX1 XXK/R, preferably a 10-amino acids cyclic peptide, having sequence XX1XXK/RXX1XXK/R, wherein X is any amino acid except lysin, arginine, histidine, tryptophane and asparagine, and X1 is any amino acid except histidine, tryptophane and asparagine.

The peptides according to the invention can be the following ones:

XXXXKXXXXK; XXXXRXXXXK; XXXXKXXXXR; XXXXRXXXXR; XKXXKXKXXK;
XKXXKXRXXK; XRXXKXKXXK; XRXXKXRXXK; XKXXRXKXXK; XRXXRXKXXK;
XKXXRXRXXK; XRXXRXRXXK; XKXXKXKXXR; XRXXKXKXXR; XKXXKXRXXR;
XRXXKXRXXR; XKXXRXKXXR; XRXXRXKXXR; XKXXRXRXXR; and XRXXRXRXXR;

wherein X is as defined above. The peptide is preferably cyclic.

Preferably, A is one of the following peptides XXXXKXXXXK, XXXXRXXXXK, XXXXKXXXXR and XXXXRXXXXR, wherein X is as defined above. The peptide is preferably cyclic. Advantageously, the invention relates to the compound as defined above, wherein the 10-amino acids peptide is preferably cyclic, and/or preferably has a sequence as set force in SEQ ID NO: 1 to SEQ ID NO: 40.

The sequences are, when the peptide is linear:

SEQ ID NO: 1 - AAPGKAAPGK;
SEQ ID NO: 2 - AAPGKAAPGR;
SEQ ID NO: 3 - AAPGRAAPGK; and
SEQ ID NO: 4 - AAPGRAAPGR.

Cyclic peptides above mentioned can be represented as follow: SEQ ID NO: 5:

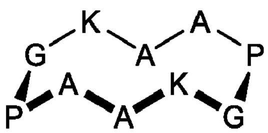

;

SEQ ID NO: 6:

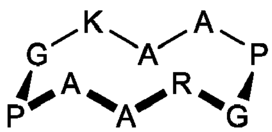

;

SEQ ID NO: 7:

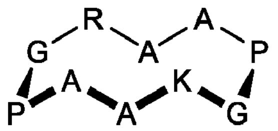 ;

SEQ ID NO: 8:

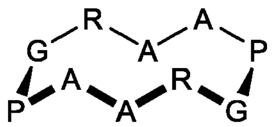 .

Other advantageous A groups are the following ones:

- AKPGKAKPGK (SEQ ID NO: 9) and the corresponding cyclic peptide SEQ ID NO: 10

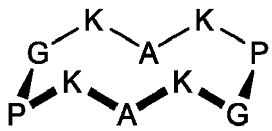 ;

- ARPGKAKPGK (SEQ ID NO: 11) and the corresponding cyclic peptide SEQ ID NO: 12

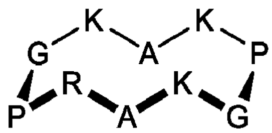 ;

- AKPGKARPGK (SEQ ID NO: 13) and the corresponding cyclic peptide SEQ ID NO: 14

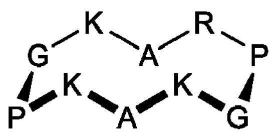 ;

- ARPGKARPGK (SEQ ID NO: 15) and the corresponding cyclic peptide SEQ ID NO: 16

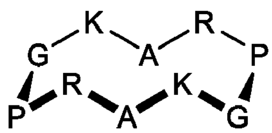 ;

- AKPGKAKPGR (SEQ ID NO: 17) and the corresponding cyclic peptide SEQ ID NO: 18

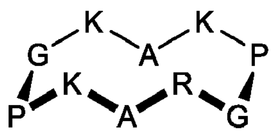 ;

- ARPGKAKPGR (SEQ ID NO: 19) and the corresponding cyclic peptide SEQ ID NO: 20

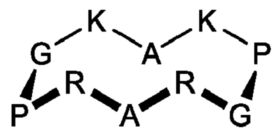 ;

- AKPGKARPGR (SEQ ID NO: 21) and the corresponding cyclic peptide SEQ ID NO: 22

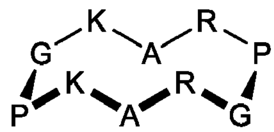 ;

- ARPGKARPGR (SEQ ID NO: 23) and the corresponding cyclic peptide SEQ ID NO:

24 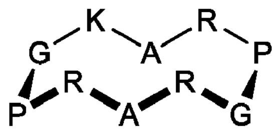 ;

- AKPGRAKPGK (SEQ ID NO: 25) and the corresponding cyclic peptide SEQ ID NO: 26

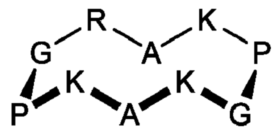 ;

- ARPGRAKPGK (SEQ ID NO: 27) and the corresponding cyclic peptide SEQ ID NO: 28

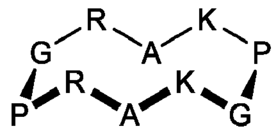 ;

- AKPGRARPGK (SEQ ID NO: 29) and the corresponding cyclic peptide SEQ ID NO: 30

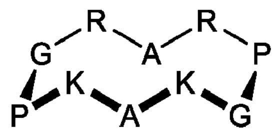 ;

- ARPGRARPGK (SEQ ID NO: 31) and the corresponding cyclic peptide SEQ ID NO:

32 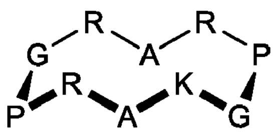 ;

- AKPGRAKPGR (SEQ ID NO: 33) and the corresponding cyclic peptide SEQ ID NO: 34

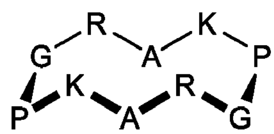 ;

- ARPGRAKPGR (SEQ ID NO: 35) and the corresponding cyclic peptide SEQ ID NO:

36 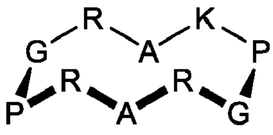 ;

- AKPGRARPGR (SEQ ID NO: 37) and the corresponding cyclic peptide SEQ ID NO:

38 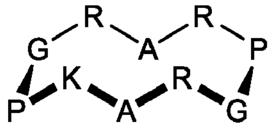 ; and

- ARPGRARPGR (SEQ ID NO: 39) and the corresponding cyclic peptide SEQ ID NO:

40 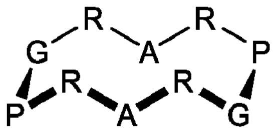 .

More advantageously, the invention relates to the compound as defined above, wherein the compound has one of the following formulas:

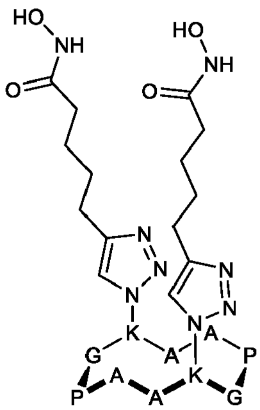

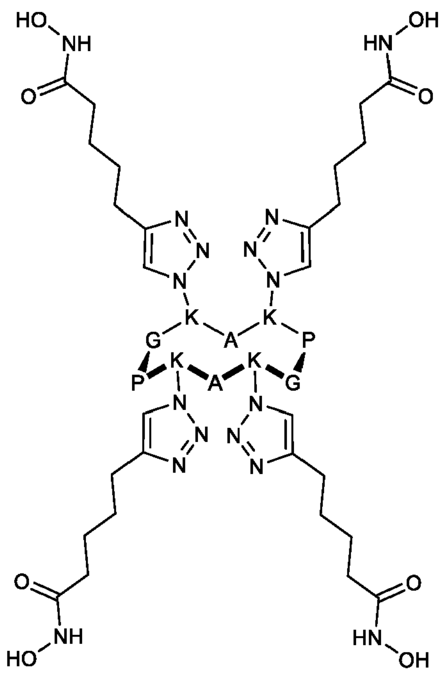 ;

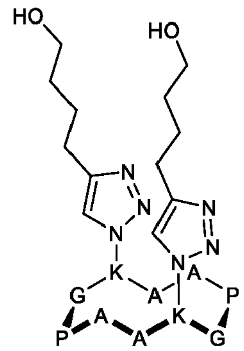

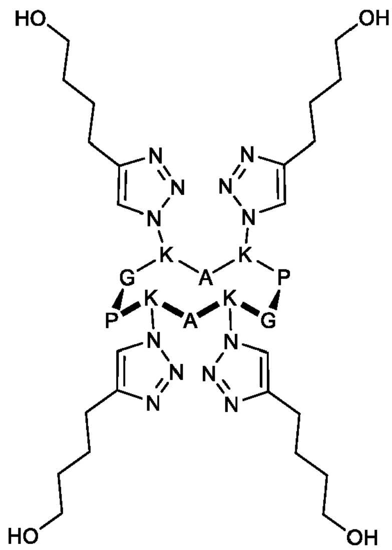 ;

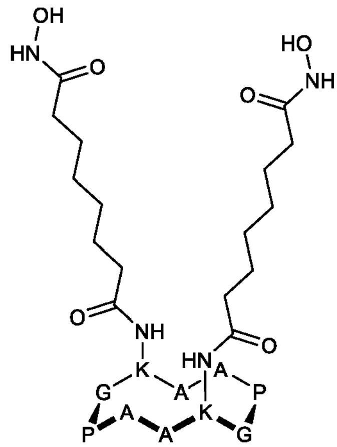 ;

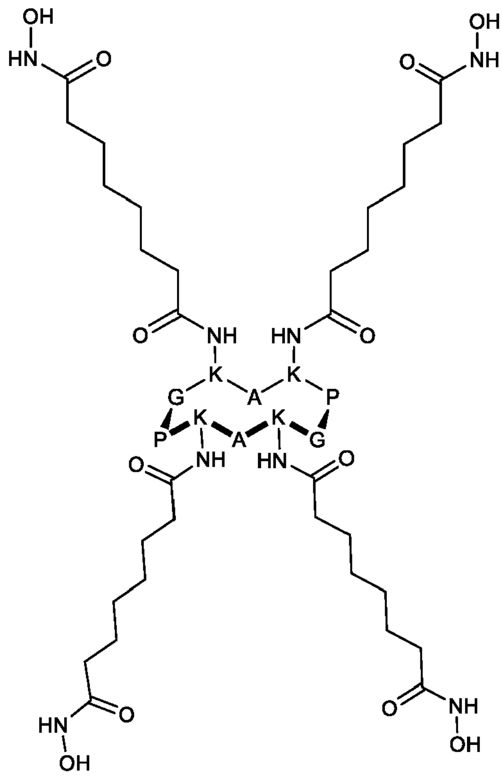 ;

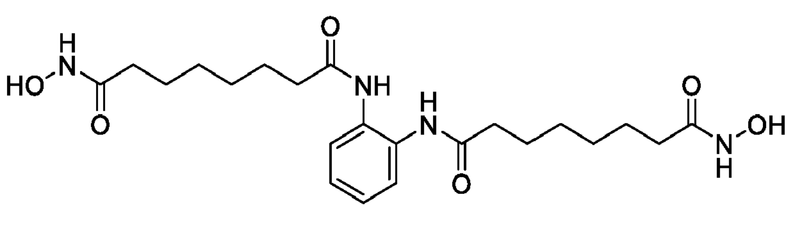 ;

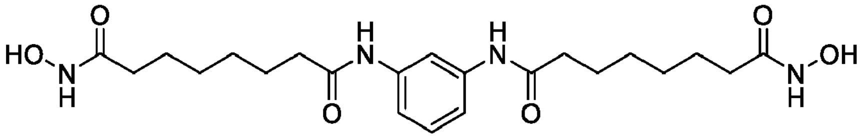

;

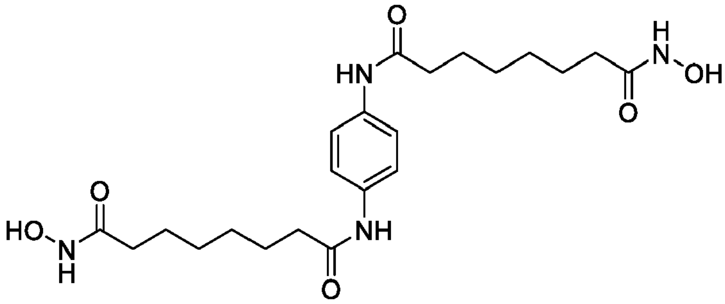

;

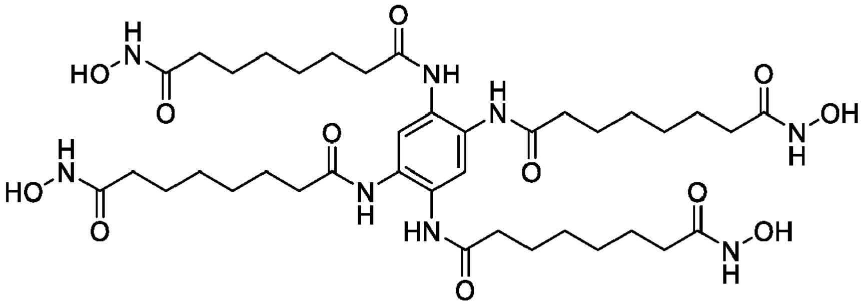

;

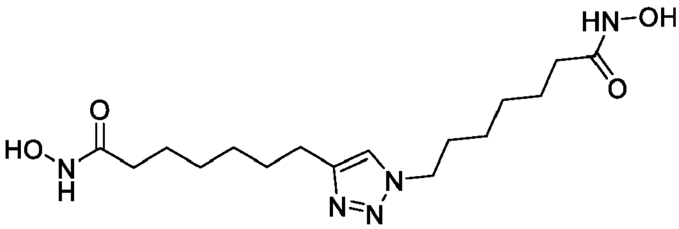

;

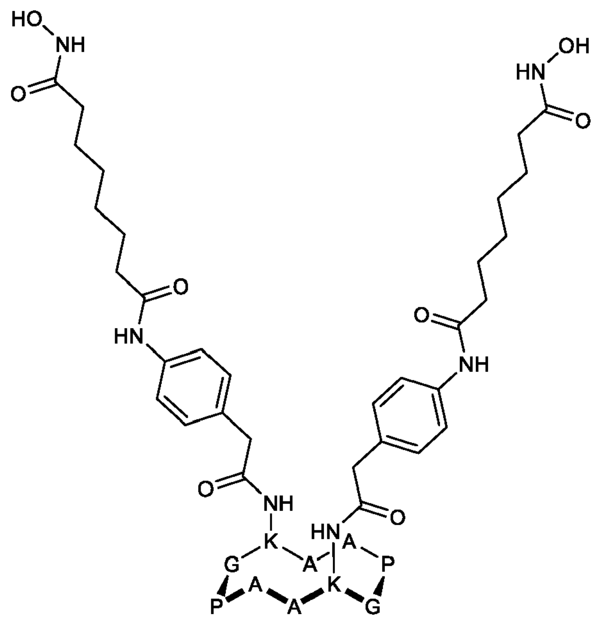

;

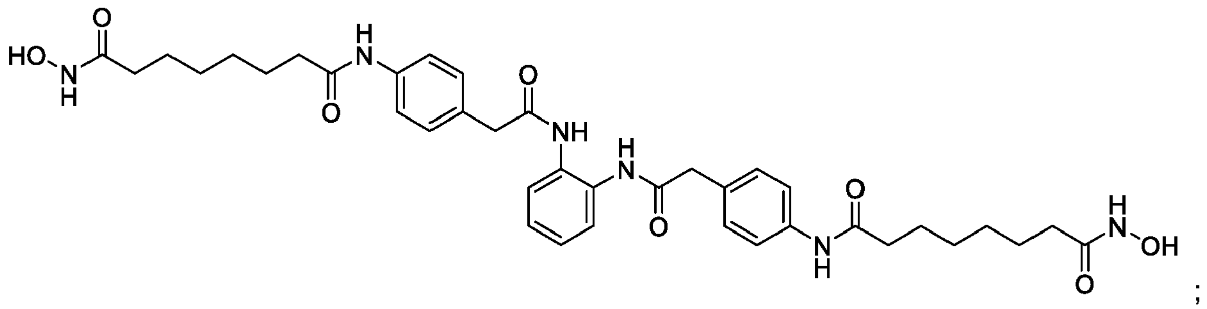

;

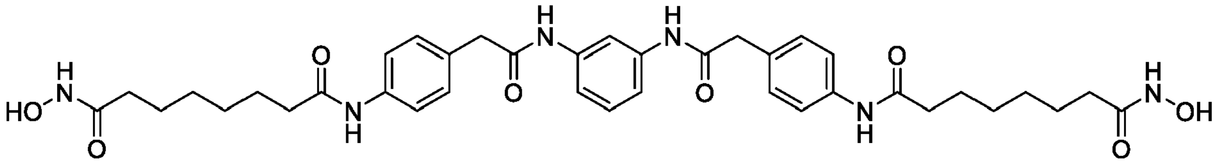

; and

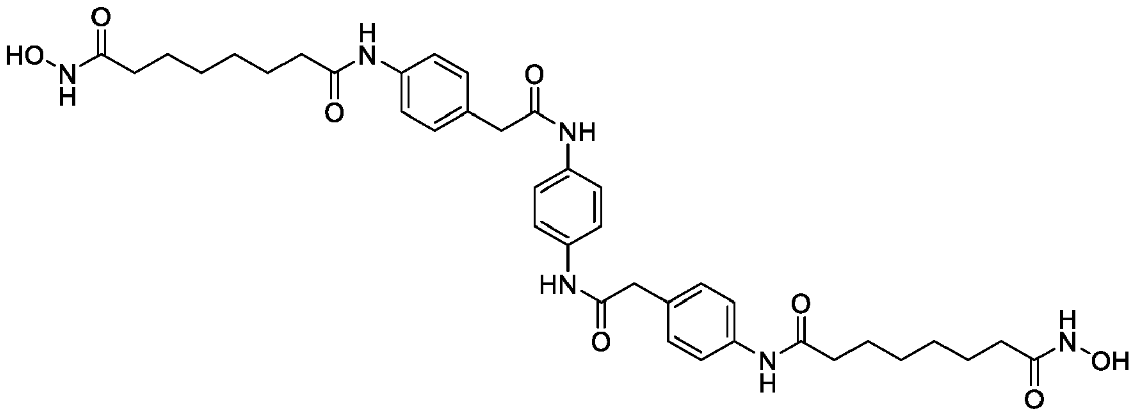

Preferably, when A is a cyclic peptide, R residues are in "cis conformation", *i.e*. R residues are oriented both either above, or below the peptide plan.

All the compounds as defined above can be also in a form of an appropriated salt, or a solvate. The invention also relates to a composition comprising as active substance the compound according to the above definition, in association with a pharmaceutically acceptable carrier. Preferably, the invention relates to a composition comprising as active substance one of the following compounds:

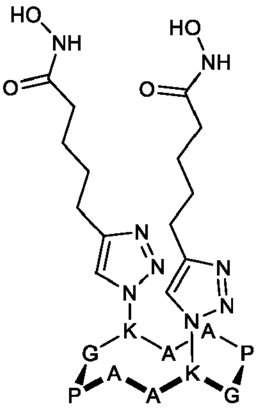
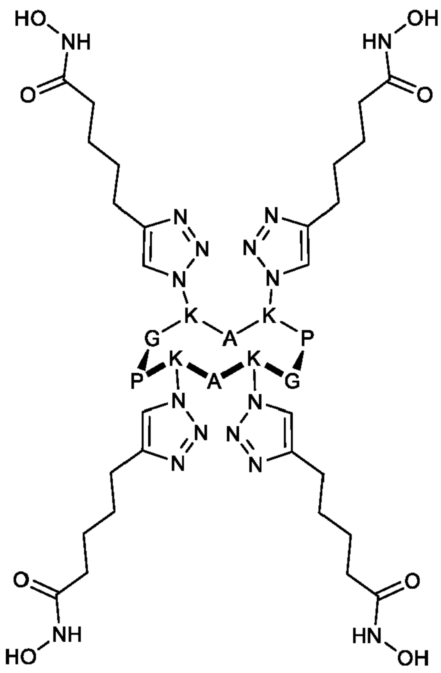
;
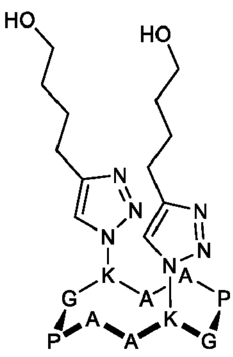
;

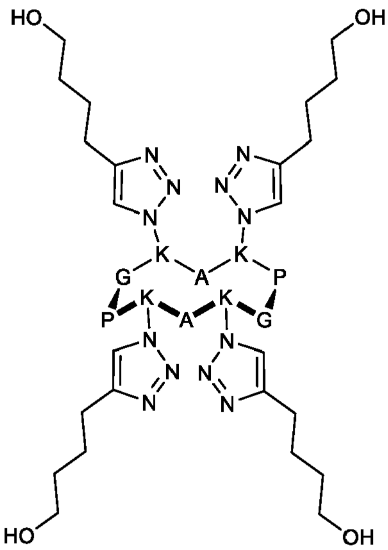
;
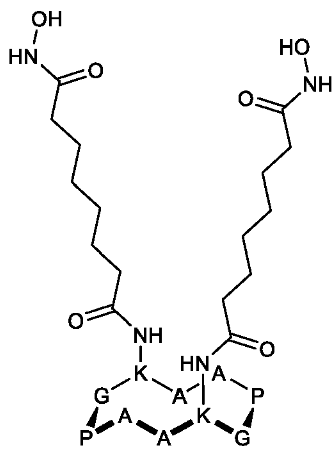
;

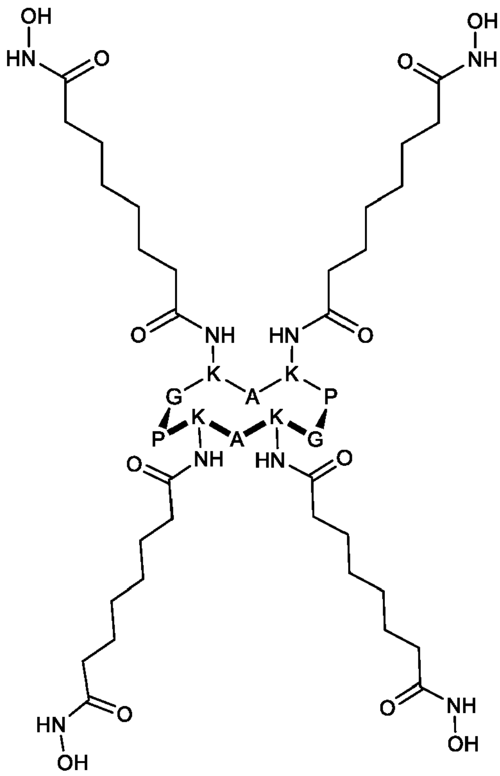

;

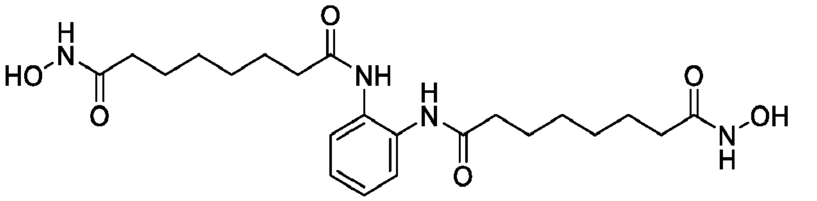

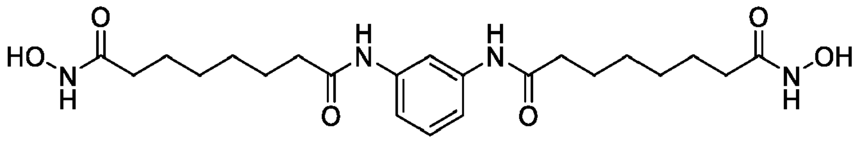

;

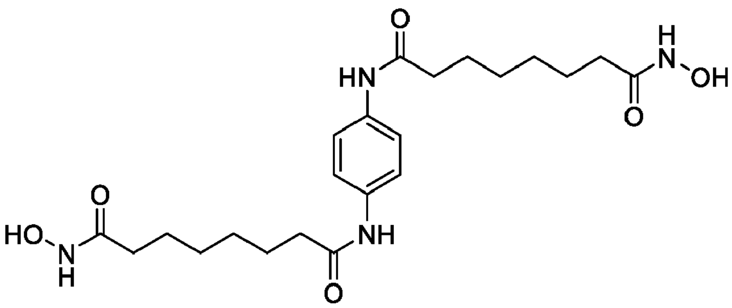

;

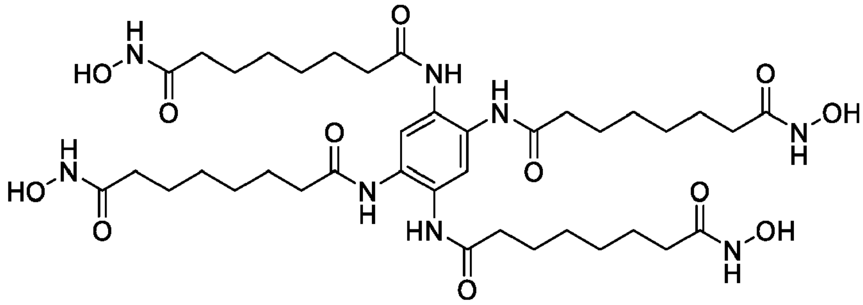

;

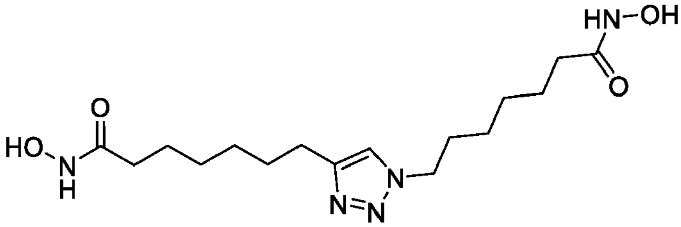

; ;

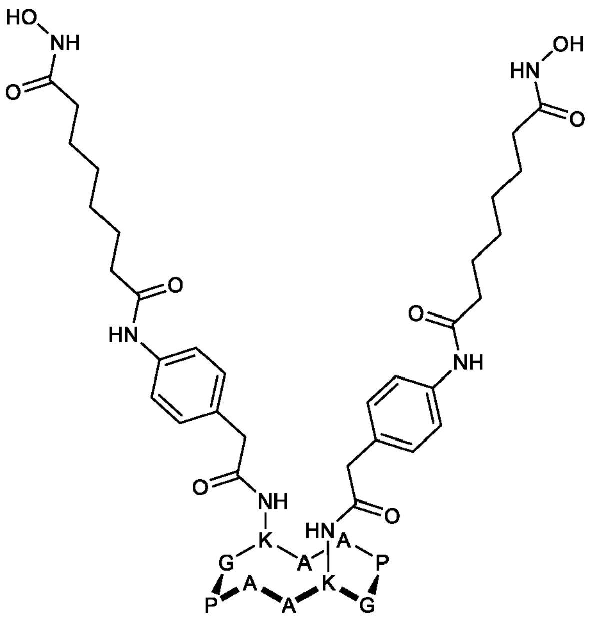

;

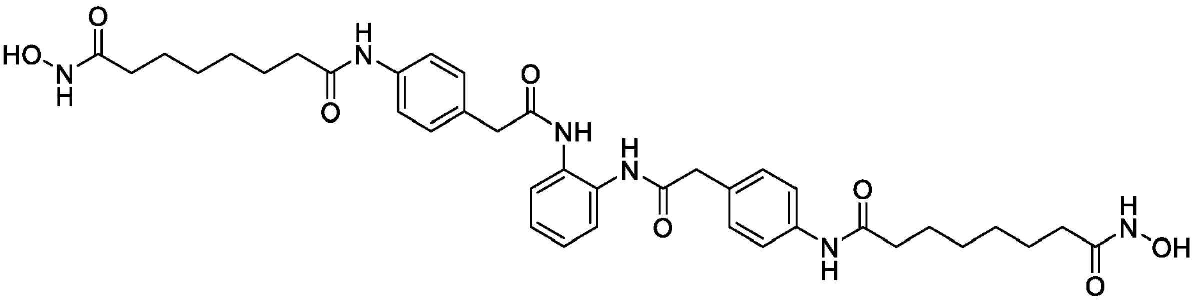

;

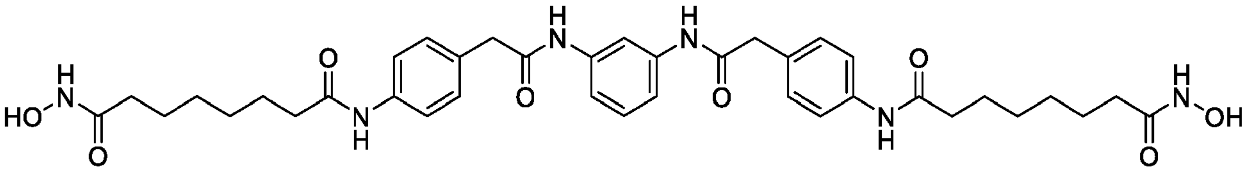

; and

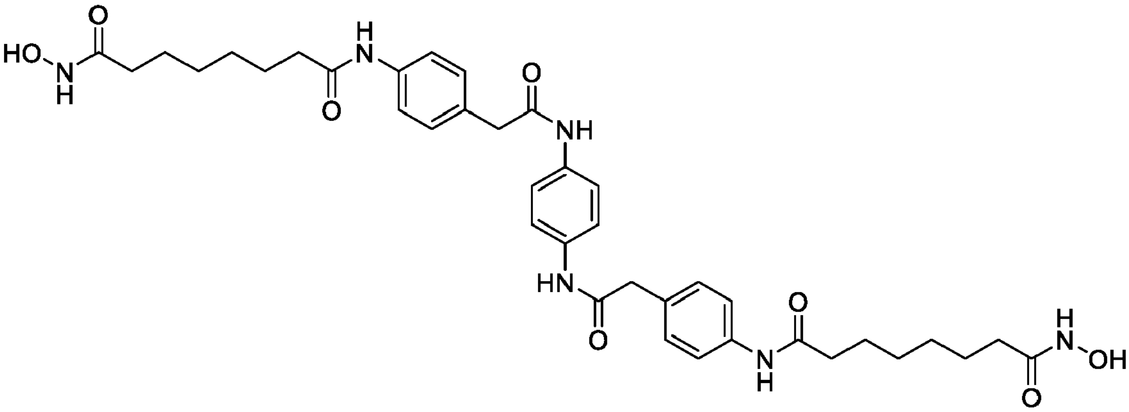

, in association with a pharmaceutically acceptable carrier.

The invention relates to the compound as defined above, for is use as medicament.

The medicament containing a compound as defined above, can be provided in any galenic form suitable for administration in humans or animals. For instance, the medicament of the present invention can exist as multi-unit or single-unit formulations. The term "multi-unit" as used herein means a plurality of discrete or aggregated particles, beads, pellets, granules, tablets, or mixtures thereof, for example, without regard to their size, shape, or morphology. Single-unit formulations include, for example, tablets, caplets, and pills.

The medicament can be in a form of a pro-drug, *i.e.* in a protected form that is inactive, and become solely active when administered orally or intravenously, by action of endogenous enzyme removing the "protection".

Within the scope of the invention, a medicament according to the invention may contain one or more of the above described compounds.

Advantageously, the invention relates to the compound as defined above, for treating a pathology involving a deregulation of a Histone deacetylase or HDAC.

It is advantageous that the compound according to the invention be administered at a dose of 100 mg to 500 mg per day, preferably once daily, more preferably with food.

The invention encompasses the pharmaceutical composition for use in a method for treating a deregulation of a histone deacetylase comprising administering such a pharmaceutical composition to a subject in need of such treatment, thereby treating the deregulation. The dosage may be in the range of from about 0.001 mg per kg body weight per day to about 1 mg per kg body weight per day, about 0.001 mg per kg body weight per day to about 0.5 mg per kg body weight per day, about 0.001 mg per kg body weight per day to about 0.1 mg per kg body weight per day.

The compound may be administered by intravenous injection, by injection into tissue, intraperitoneally, orally, or nasally. The compound may have a form selected from the group consisting of a solution, dispersion, suspension, powder, capsule, tablet, pill, time release capsule, time release tablet, and time release pill.

Advantageously, the invention relates to the compound as defined above, for its use as defined above, wherein the pathology involving a deregulation of a histone deacetylase or HDAC is:

- cancer, in particular hematological cancer or solid tumor such as breast cancer, thyroid cancer, prostate cancer...,
- Crohn disease,
- schizophrenia,
- HIV infection,
- *Plasmodium falciparum* and *Schistosoma mansoni* infection
- Parkinson's and Alzheimer's diseases.

The invention also relates to a method for treating a pathology involving a deregulation of a histone deacetylase or HDAC, the method comprising administering an effective amount of a compound as defined above to a patient in a need

thereof.

The invention also relates to a method for treating cancer, the method comprising administering an effective amount of a compound as defined above to a patient in a need thereof.

The invention also relates to a method for treating Crohn disease, the method comprising administering an effective amount of a compound as defined above to a patient in a need thereof.

The invention also relates to a method for treating schizophrenia, the method comprising administering an effective amount of a compound as defined above to a patient in a need thereof.

The invention also relates to a method for treating Parkinson and / or Alzheimer's diseases, the method comprising administering an effective amount of a compound as defined above to a patient in a need thereof.

The invention also relates to a compound of formula $A(R)_4$ wherein at least 2R are benzamine derivative of formula II

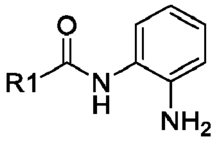 (II),

wherein R1 is a C1-C6 alkyl, linear or branched, saturated or not; an aryl, possibly substituted by an alkyl amide, an alkyl diamide
and wherein A is as defined above.

Advantageously, the invention relates to the compound according to the above definition, wherein A is

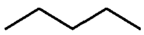 ; 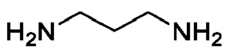 ; 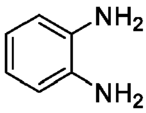 ; 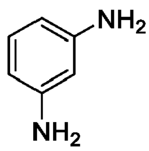 ; 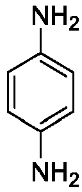 ; 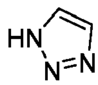

or a 10-amino acids peptide having a sequence as set force in the following sequence XX1XXK/RXX1XXK/R, preferably a 10-amino acids cyclic peptide, having sequence XX1XXK/RXX1XXK/R, wherein X is any amino acid except lysin, arginine, histidine, tryptophane and asparagine, and X1 is any amino acid except histidine, tryptophane and asparagine.

The peptides according to the invention can be the following ones:

XXXXKXXXXK; XXXXRXXXXK; XXXXKXXXXR; XXXXRXXXXR; XKXXKXKXXK;
XKXXKXRXXK; XRXXKXKXXK; XRXXKXRXXK; XKXXRXKXXK; XRXXRXKXXK;
XKXXRXRXXK; XRXXRXRXXK; XKXXKXKXXR; XRXXKXKXXR; XKXXKXRXXR;
XRXXKXRXXR; XKXXRXKXXR; XRXXRXKXXR; XKXXRXRXXR; and XRXXRXRXXR;

wherein X is as defined above. The peptide is preferably cyclic.

Preferably, A is one of the following peptides XXXXKXXXXK, XXXXRXXXXK, XXXXKXXXXR and XXXXRXXXXR, wherein X is as defined above. The peptide is preferably cyclic.

Advantageously, the invention relates to the compound as defined above, wherein the 10-amino acids peptide is preferably cyclic, and/or preferably has a sequence as set force in SEQ ID NO: 1 to SEQ ID NO: 40.

The sequence is, when the peptide is linear:

SEQ ID NO: 1 - AAPGKAAPGK;
SEQ ID NO: 2 - AAPGKAAPGR;
SEQ ID NO: 3 - AAPGRAAPGK; and
SEQ ID NO: 4 - AAPGRAAPGR.

Cyclic peptides above mentioned can be represented as follow: SEQ ID NO: 5:

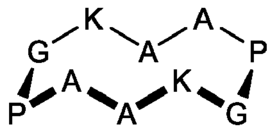 ;

SEQ ID NO: 6:

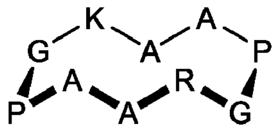 ;

SEQ ID NO: 7:

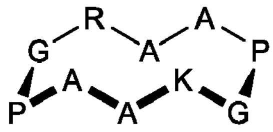 ;

SEQ ID NO: 8:

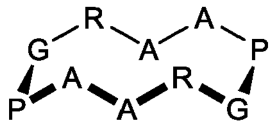 .

Other advantageous A groups are the following ones:

- AKPGKAKPGK (SEQ ID NO: 9) and the corresponding cyclic peptide SEQ ID NO: 10

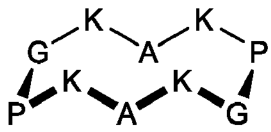 ;

- ARPGKAKPGK (SEQ ID NO: 11) and the corresponding cyclic peptide SEQ ID NO: 12

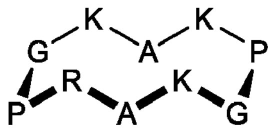 ;

- AKPGKARPGK (SEQ ID NO: 13) and the corresponding cyclic peptide SEQ ID NO: 14

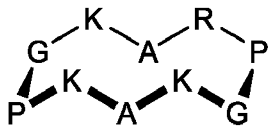 ;

- ARPGKARPGK (SEQ ID NO: 15) and the corresponding cyclic peptide SEQ ID NO: 16

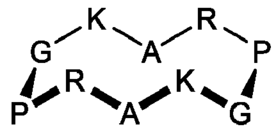

;

- AKPGKAKPGR (SEQ ID NO: 17) and the corresponding cyclic peptide SEQ ID NO: 18

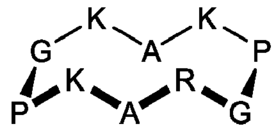

;

- ARPGKAKPGR (SEQ ID NO: 19) and the corresponding cyclic peptide SEQ ID NO: 20

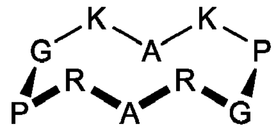

;

- AKPGKARPGR (SEQ ID NO: 21) and the corresponding cyclic peptide SEQ ID NO: 22

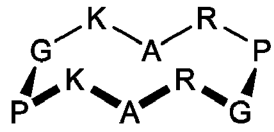

;

- ARPGKARPGR (SEQ ID NO: 23) and the corresponding cyclic peptide SEQ ID NO: 24

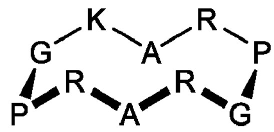

;

- AKPGRAKPGK (SEQ ID NO: 25) and the corresponding cyclic peptide SEQ ID NO: 26

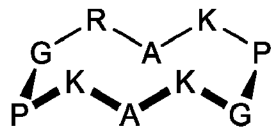

;

- ARPGRAKPGK (SEQ ID NO: 27) and the corresponding cyclic peptide SEQ ID NO: 28

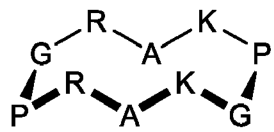

;

- AKPGRARPGK (SEQ ID NO: 29) and the corresponding cyclic peptide SEQ ID NO: 30

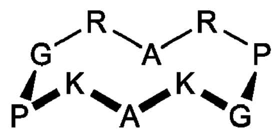

;

- ARPGRARPGK (SEQ ID NO: 31) and the corresponding cyclic peptide SEQ ID NO: 32

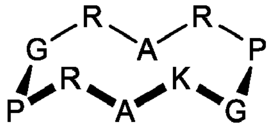
;

- AKPGRAKPGR (SEQ ID NO: 33) and the corresponding cyclic peptide SEQ ID NO: 34

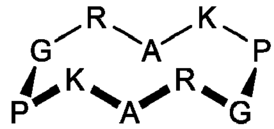
;

- ARPGRAKPGR (SEQ ID NO: 35) and the corresponding cyclic peptide SEQ ID NO: 36

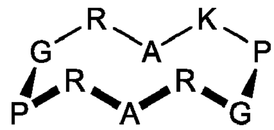
;

- AKPGRARPGR (SEQ ID NO: 37) and the corresponding cyclic peptide SEQ ID NO: 38

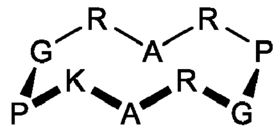
;

and ARPGRARPGR (SEQ ID NO: 39) and the corresponding cyclic peptide SEQ ID NO: 40

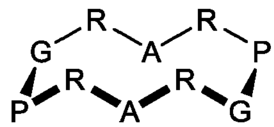
.

More advantageously, the invention relates to the compound as defined above, wherein R is chosen from:

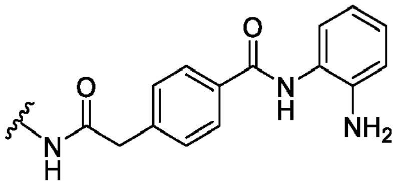
;

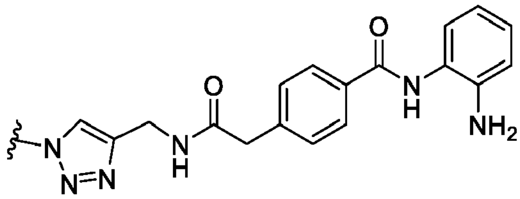

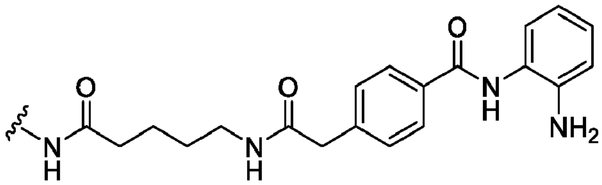

; and

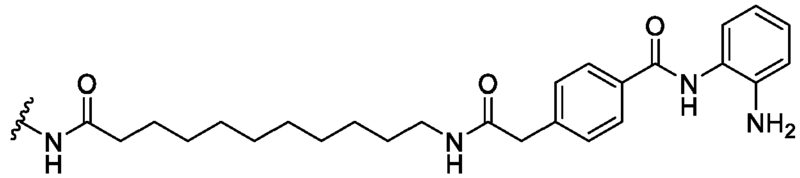

.

More advantageously, the invention relates to the compound according to the above definition, wherein the compound has one of the following formulas:

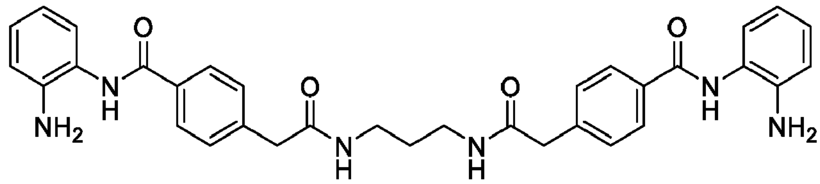

;

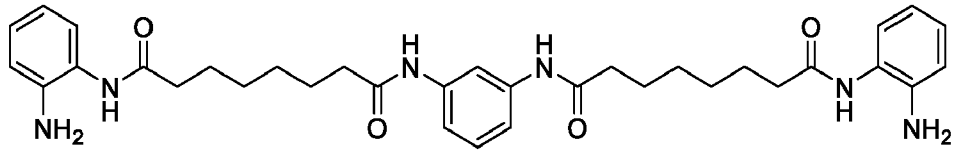

;

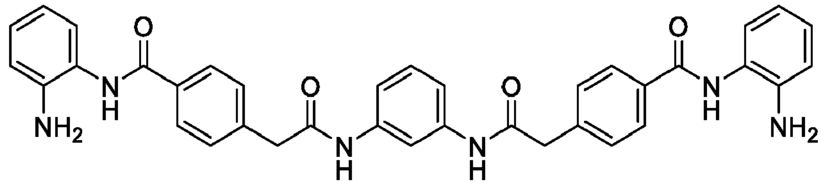

;

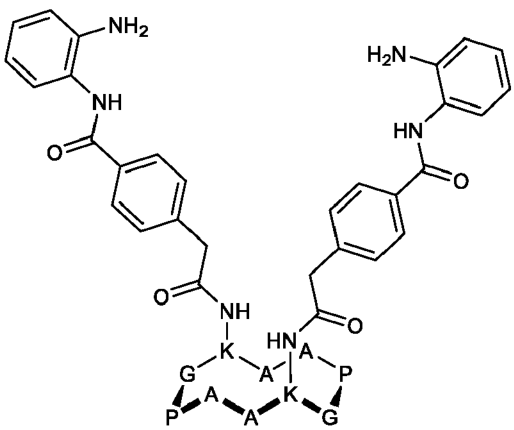

;

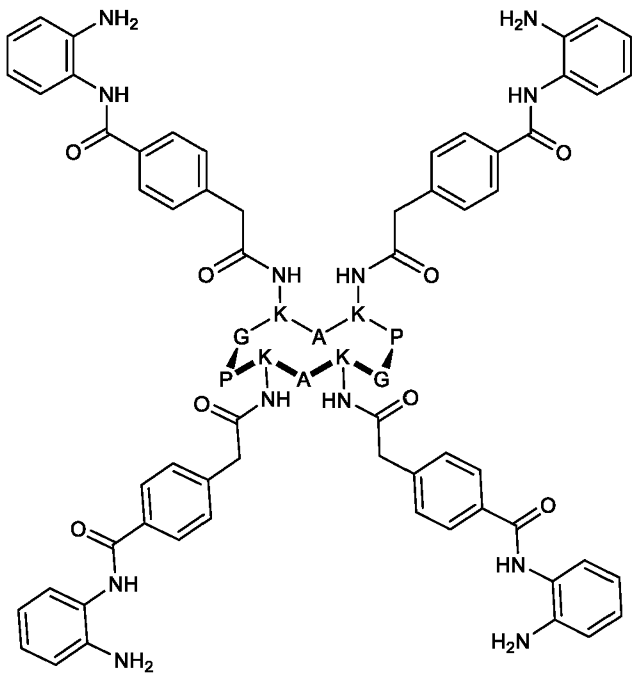

;

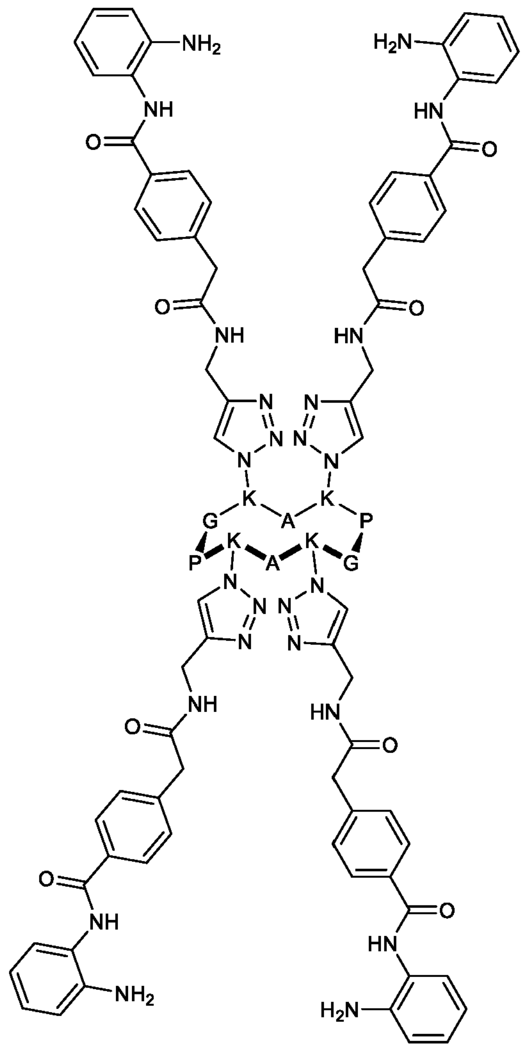

;

and

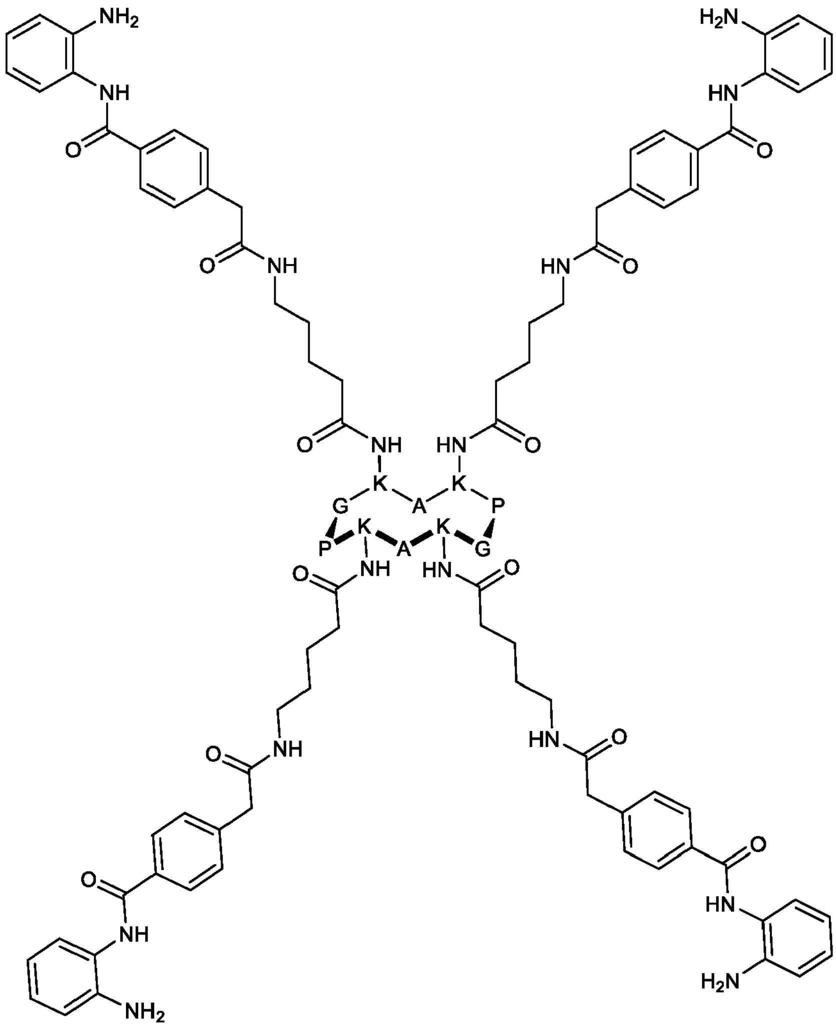

Preferably, when A is a cyclic peptide, R residues are in "*cis* conformation", *i.e*. R residues are oriented both either above, or below the peptide plan.

Advantageously, the invention relates to a pharmaceutical composition comprising as active substance the compound according as defined above, in association with a pharmaceutically acceptable carrier.

The invention also relates to the compound or the pharmaceutical composition as defined above, for is use as medicament.

More advantageously, the invention relates to the compound as defined above, for treating a pathology involving a deregulation of a histone deacetylase or HDAC.

More advantageously, the invention relates to the compound for its use according to claim 10, wherein the pathology involving a deregulation of a Histone deacetylase or HDAC is:

- cancer, in particular hematological cancer or solid tumor such as breast cancer, thyroid cancer, prostate cancer...,
- Crohn disease,
- schizophrenia,
- HIV infection,
- *Plasmodium falciparum* and *Schistosoma mansoni* infection
- Parkinson and Alzheimer's diseases.

The invention also relates to a method for treating a pathology involving a deregulation of a histone deacetylase or HDAC, the method comprising administering an effective amount of a compound as defined above to a patient in a need thereof.

The invention also relates to a method for treating cancer, the method comprising administering an effective amount of a compound as defined above to a patient in a need thereof.

The invention also relates to a method for treating Crohn disease, the method comprising administering an

effective amount of a compound as defined above to a patient in a need thereof.

The invention also relates to a method for treating schizophrenia, the method comprising administering an effective amount of a compound as defined above to a patient in a need thereof.

The invention also relates to a method for treating Parkinson and / or Alzheimer's diseases, the method comprising administering an effective amount of a compound as defined above to a patient in a need thereof.

**Brief description of the drawings**

The invention will be better understood in view of the following examples and the following drawings:

[Fig. 1] is a representation of the synthesis scheme of the cyclic peptides used in the invention.
[Fig. 2] is a representation of the synthesis scheme of cyclic peptides wherein azide group(N3) is added to the lysine residues.

**Examples**

All reagents and solvents were purchased from commercial suppliers, Sigma-Aldrich, TCI or Alfa-Aesar. All reactions were monitored by TLC on silica gel 60 $F_{254}$ plates (Merck Art. No. 1.05554). Purifications by flash chromatography were carried out on a Puriflash 430 instrument (Interchim) using 30 μm spheric silica or 40-60 μm irregular silica cartridges. Preparative and semi-preparative HPLC were performed on an VWR instrument equipped with a LaPrep P110 pump and LaPrep P314 Dual absorbance detector. A C18 reversed-phase column (Waters, XSelect CSH C18 OBD, 30 x 250 mm, 5 μm) was used for preparative HPLC and C18 reversed-phase column (Macherey-Nagel, Nucleodur C18 HTec, 21 × 250 mm, 7 μm) was used for semi-preparative HPLC. Elution was performed using a binary gradient (solvent A: $H_2O$ + 0.1% TFA; solvent B: ACN + 0.1% TFA). Compound purity and stability were measured by high-performance liquid chromatography (HPLC, Thermo Scientific, UltiMate3000) on C18 reversed-phase column (Thermo Scientific, Hypersil GOLD aQ, 2.1 × 50 mm, 1.9 μm). Elution was performed using a binary gradient (solvent A: $H_2O$ + 0.1% TFA; solvent B: ACN + 0.1% TFA). Low resolution mass spectra were acquired using electrospray in positive and negative modes. All MS analysis samples were prepared as solutions in ACN. LCMS analyses were performed on an instrument equipped with a Shimadzu LC-20AD XR pump and a SPD-M20A diode array detector. A C18 reversed-phase column (Phenomenex, Kinetex polar C18, 3 x 750 mm, 2.6 μm, 100 Å) was used. Elution was performed using a binary gradient (solvent A: $H_2O$ + 0.1% formic acid; solvent B: ACN + 0.1% formic acid). Final compounds were analysed by high-resolution ESI mass spectrometry (HRMS) using a microTOF-Q mass spectrometer fitted with an electrospray ion source. NMR spectra were recorded on a Bruker DRX-400 spectrometer at 400 MHz for $^1H$ and 101 MHz for $^{13}C$. 2D NMR experiments such as $^1H$-$^1H$ COSY, $^1H$-$^{13}C$ HSQC, $^1H$-$^{13}C$ HMBC experiments were performed to enable signal attributions. Chemical shifts ($\delta$) are given in ppm, coupling constant (*J*) are measured in hertz (Hz). Multiplicity is indicated as follows: s, singlet; d, doublet; t, triplet; q, quadruplet; dd, doublet of doublet; ddd, doublet of doublet of doublet; m, multiplet.

Example 1: Synthesis of hydoxamic acid-based multivalent compounds involving aromatic and alkyl platforms

1- di or tetravalent compounds

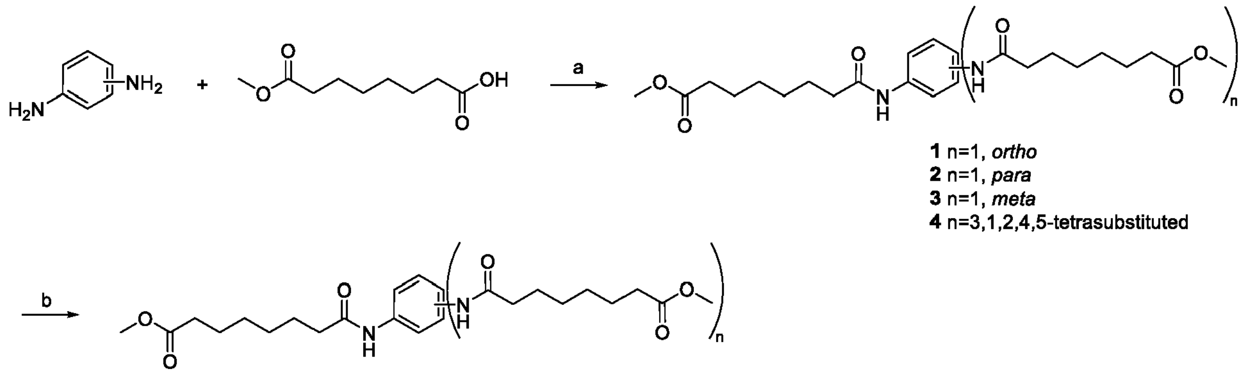


**1** n=1, *ortho*
**2** n=1, *para*
**3** n=1, *meta*
**4** n=3,1,2,4,5-tetrasubstituted

**MuH2322** n=1, *ortho*
**MuH2323** n=1, *para*
**MuH2323** n=1, *meta*
**MuH2344** n=3,1,2,4,5-tetrasubstituted

**(a)** EDCI.HCl, DMAP, DCM or HATU, DIPEA, DMF, RT, 18 h; **(b)** $NH_2OH$.HCl, KOH, MeOH, RT, 1 h to 3 h

2- Divalent compounds 2.1

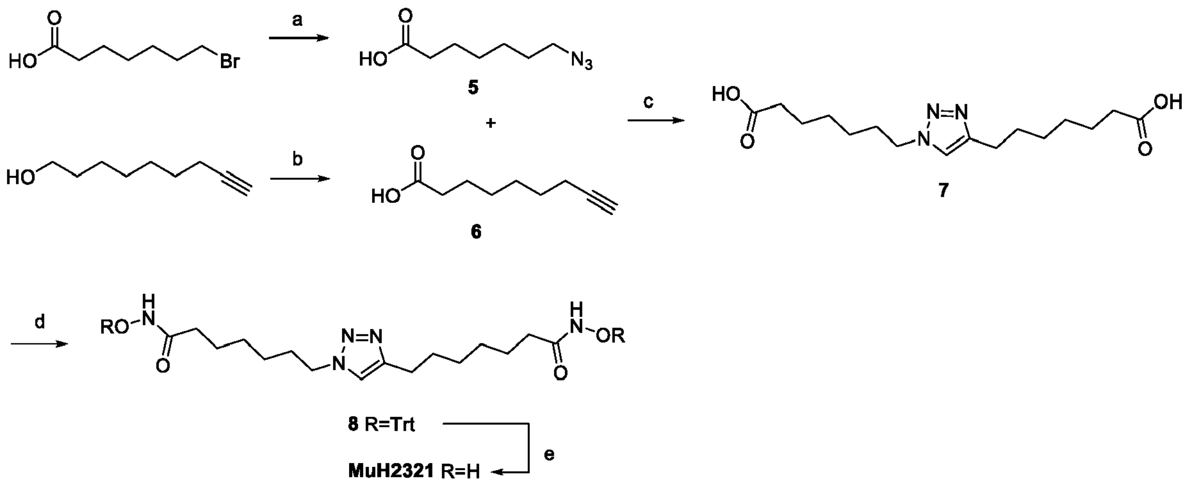


**5**
**6**
**7**
**8** R=Trt
**MuH2321** R=H

**(a)** $NaN_3$, DMF, reflux, 16 h; **(b)** $CrO_3$, $H_2SO_4$, $H_2O$, Acetone, 0 °C to RT, 3 h; **(c)** $Cu(OAc)_2$, NaAsc, tBuOH/$H_2O$, RT, 2 h; **(d)** $NH_2OTrt$, HATU, DIPEA, DMF, RT, 16 h; **(e)** TFA, $CH_2Cl_2$, Tis, RT, 30 min.

**General procedure A: monomethylsuberate coupling on tetra/diaminebenzene**

Monomethyl suberate (1.1 eq. by amine) was solubilized in dry $CH_2Cl_2$ [0.4 M], then EDCI.HCl (1.25 eq. by amine) was added, followed by DMAP (0.1 eq. by amine) and the appropriate diamine (1 eq.). The reaction mixture was stirred overnight at room temperature and monitored by TLC. After dilution with $H_2O$ and the mixture was extracted with $CH_2Cl_2$ three times. The organic phases were washed with saturated $NaHCO_3$ solution and brine and then, dried over $Na_2SO_4$, filtered, and concentrated under vacuum. The compound was purified by flash chromatography using a linear gradient ($CH_2Cl_2$/MeOH: 100/0 to 95/05).

**General procedure B: hydroxamic acid from monomethyl ester**

Hydroxyamine hydrochloride (5 eq. by amine) and potassium hydroxide (5 eq. by amine) were solubilized in MeOH (7.7 ml) and stirred at 40 °C. After 1 h the precipitate was filtered of, and the appropriate methyl ester compound (1 eq.) was added to the mixture. Additional potassium hydroxide was added (until pH= 10-12) and the reaction mixture was stirred at room temperature until completion of the reaction. After 1 h, the mixture was acidified to pH= 5-6 with HCl 1N and filtered to afford the product as a powder.

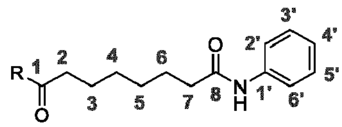

**Dimethyl 8,8'-(1,2-phenylenebis(azanediyl))bis(8-oxooctanoate). (1)**

Following the general procedure A, compound **1** was obtained as a white powder (593.9 mg, 1.32 mmol, y. 72%). **R*f*=** 0.3 ($CH_2Cl_2$/MeOH 97/3). **$^1$H NMR** (400 MHz, $CDCl_3$): δ (ppm) 8.53 (br, 2H, NH), 7.26 (m, 2H, H-3'/6'), 7.11 (m, 2H, H-4'/5'), 3.65 (s, 6H, $CH_3$ methyl ester) 2.30 (t, J = 7.5 Hz, 4 H, H-7), 2.25 (t, J = 7.6 Hz, 4H, H-2), 1.74 - 1.49 (m, 8H, H-3/6), 1.43 - 1.26 (m, 8H, H-4/5). **$^{13}$C NMR** (101 MHz, $CDCl_3$): δ (ppm) = 174.2 (C-1), 172.8 (C-8), 130.6 (C-1'/2'), 126.0 (C-3'/6'), 125.5 (C-4'/5'), 51.5 ($CH_3$ methyl ester), 36.9 (C-2), 34.0 (C-7), 28.9, 28.8 (C-4/5), 25.5, 24.8 (C-3/6). **ESI-MS** *m*/*z*=449.3 $[M+H]^+$, 897.6 $[2M+H]^+$.

***N$^1$*,*N$^{1'}$*-(1,2-Phenylene)bis(N$^8$-hydroxyoctanediamide). (MuH2322)**

Following general procedure B from compound **1** (561.3 mg, 1.23 mmol). The mixture was acidified to pH=1 with HCl 1N and filtered. The solid was dried to afford **MuH2322** as a white powder (392.1 mg, 8.7 mmol, y. 70 %). **$^1$H NMR** (400 MHz, DMSO): δ (ppm) = 10.35 (br, 2H, OH), 9.29 (br, 2H, NH), 7.49 (dd, J = 6.1, 3.6 Hz, 2H, H-3'/6'), 7.13 (dd, J = 6.1, 3.6 Hz, 2H, H-4'/5'), 2.31 (t, J = 7.4 Hz, 4H, H-7), 1.94 (t, J = 7.4 Hz, 4H, H-2), 1.58 (p, J = 7.4 Hz, 4H, H-6), 1.49 (p, J = 7.3 Hz, 4H, H-3), 1.35 - 1.05 (m, 8H, H-4/5). **$^{13}$C NMR** (101 MHz, DMSO): δ (ppm) = 171.5 (C-8), 169.2 (C-1), 130.6 (C-1'/2'), 124.9 (C-3'/6'), 124.8 (C-4'/5'), 36.1 (C-7), 32.3 (C-2), 28.4 (C-4/5), 25.07 (C-3/6). **HRMS** Calcd for $C_{22}H_{35}N_4O_6$ 451.2551, Found 451.2552.

**Dimethyl 8,8'-(1,4-phenylenebis(azanediyl))bis(8-oxooctanoate). (2)**

Following the general procedure, compound **2** was obtained as a white solid (292.2 mg, 0.66 mmol, y. 33%). **R*f*=** 0.23 ($CH_2Cl_2$/MeOH 95/5). **$^1$H NMR** (400 MHz, $CDCl_3$): δ (ppm) = 7.45 br, 2H, NH), 7.27 (s, 4H, H-2'/3'/5'/6'), 3.66 (s, 6H, $CH_3$-Me), 2.39 - 2.26 (m, 8H, H-2/7), 1.72 (p, J = 7.2 Hz, 4H; H-6), 1.64 (p, J = 7.2 Hz, 4H, H-3), 1.36 (m, 8H, H-4/5). **ESI-MS** *m*/*z*=449.2 $[M+H]^+$, 897.5 $[2M+H]^+$.

***N$^1$*,*N$^{1'}$*-(1,4-Phenylene)bis(N8-hydroxyoctanediamide). (MuH2323)**

Following general procedure B from compound **2** (212.4 mg, 0.47 mmol). **MuH2323** was obtained as white powder (134.8 mg, 0.30 mmol, y. 64 %). **$^1$H NMR** (400 MHz, DMSO): δ (ppm) = 9.82 (br, 2H, NH), 7.49 (s, 4H, H-2'/3'/5'/6'), 2.27 (t, J = 7.4 Hz, 4H, H-7), 1.94 (t, J = 7.4 Hz, 4H, H-2), 1.57 (q, J = 7.4 Hz, 4H, H-6), 1.49 (q, J = 7.4 Hz, 4H, H-3), 1.27 (d, J = 6.2 Hz, 8H, H-4/5). **$^{13}$C NMR** (101 MHz, DMSO): δ (ppm) = 170.9 (C-8), 169.1 (C-1), 134.6 (C-1'/4'), 119.4 (C-2'/3'/5'/6'), 36.3 (C-7), 32.2 (C-2), 28.4 (C-4/5), 25.1 (C-3/6). **HRMS** Calcd for $C_{22}H_{35}N_4O_6$ 451.2551, Found 451.2560.

**Dimethyl 8,8'-(1,3-phenylenebis(azanediyl))bis(8-oxooctanoate).** (3)

Following the general procedure A, compound **3** was obtained as a red solid (351.6 mg, 0.78 mmol, y. 42%). **R*f*=** 0.37 ($CH_2Cl_2$/MeOH 97/3). **$^1$H NMR** (400 MHz, $CDCl_3$): δ (ppm) = 7.81 - 7.75 (s, 1H, H-2'), 7.73 (br, 2H, NH), 7.27 (d, J = 8.3 Hz, 2H, H-4'/6'), 7.23 - 7.15 (t, J = 8.3 Hz, 1H, H-5'), 3.65 (s, 6H, $CH_3$-Me), 2.29 (m, 8H, H-7/2), 1.67 (p, J = 7.3 Hz, 4H, H-6), 1.64 - 1.52 (p, J = 7.3 Hz 4H, H-3), 1.38 - 1.26 (m, 8H, H-4/5). **$^{13}$C NMR** (101 MHz, $CDCl_3$): δ (ppm) = 174.4 (C-8), 172.0 (C-1), 138.7 (C-1'/3'), 129.5 (C-5'), 115.6 (C-4'/6'), 111.5 (C-2'), 51.6 (C-Me), 37.6 (C-7), 34.0 (C-2), 28.8 (C-4/5), 25.43 (C-6), 24.7 (C-3). **ESI-MS** *m*/*z*=449.2 $[M+H]^+$, 897.5 $[2M+H]^+$.

***N$^1$*,*N$^{1'}$*-(1,3-Phenylene)bis(N8-hydroxyoctanediamide). (MuH2324)**

Following general procedure B from compound **3** (292.2 mg, 0.66 mmol). **MuH2324** was obtained as an orange powder (264.9 mg, 0.58 mmol, y. 89%). **$^1$H NMR** (600 MHz, DMSO): δ (ppm) = 10.32 (br, 2H, OH), 9.83 (br, 2H, NH), 8.65 (br, 2H, NH-OH), 7.91 (t, J = 2.1 Hz, 1H, H-2'), 7.25 (dd, J = 8.1, 2.1 Hz, 2H, H-4'/6'), 7.16 (t, J = 8.1 Hz, 1H, H-5'), 2.27 (t, J = 7.4 Hz, 4H, H-7), 1.93 (t, J = 7.4 Hz, 4H, H-2), 1.56 (p, J = 7.2 Hz, 4H, H-6), 1.48 (p, J = 7.4 Hz, 4H, H-3), 1.39 - 1.08 (m, 8H, H-4/5). **$^{13}$C NMR** (151 MHz, DMSO): δ (ppm) = 171.7 (C-8), 169.6 (C-1), 140.0 (C-1'/3'), 129.2 (C-5'), 114.3 (C-4'/6'), 110.5 (C-2'), 36.8 (C-7), 32.7 (C-2), 28.9 (C-4/5), 25.5 (C-3/6). **HRMS** Calcd for $C_{22}H_{35}N_4O_6$ 451.2551, Found 451.2552.

**Tetramethyl 8,8',8'',8'''-(benzene-1,2,4,5-tetrayltetrakis(azanediyl))tetrakis(8-oxooctanoate). (4)**

Monomethyl suberate (298.2 mg, 1.58 mmol, 4.5 eq.) was solubilized in 3.5 ml of dry DMF. Then, HATU (600.0 mg, 1.58 mmol, 4.5 eq.) was added, followed by 1,2,4,5-benzenetetraamine.4HCl (102.7 mg, 0.35 mmol, 1 eq.) and DIPEA dropwise (0.5 ml, 2.82 mmol, 8 eq.). The mixture was stirred overnight at room temperature and monitored by HPLC. After completion of the reaction, the reaction mixture was diluted with $H_2O$ and extracted with $CH_2Cl_2$ three times. The organic phases were washed with $H_2O$, saturated NaHCOs solution and brine and then, dried over $Na_2SO_4$, filtered, and concentrated under *vacuum.* The compound was purified by flash chromatography using a linear gradient ($CH_2Cl_2$/MeOH: 100/0 to 95/05) to afford compound **4** as a white solid (188.4 mg, 0.23 mmol, y. 64%) **R*f*=** 0.57 ($CH_2Cl_2$/MeOH 95/5). **$^1$H NMR** (400 MHz, DMSO): δ (ppm) = 9.28 (br, 4H, NH), 7.66 (s, 2H, H-3'/6'), 3.59 (s, 12H, $CH_3$-Me), 2.38 - 2.23 (m, 16H, H-2/7), 1.81 - 1.42 (m, 16H, H-3/6), 1.31 (m, 16H, H-4/5). **$^{13}$C NMR** (101 MHz, DMSO): δ (ppm) = 173.8 (C-8), 171.9 (C-7), 127.8 (C-1'/2'/4'/5'), 121.1 (C-3'/6'), 51.6 (C-Me), 36.5 (C-7), 33.7 (C-2), 28.8 (C-4/5), 25.4 (C-6), 24.8 (C-3). **ESI-MS** m/z=819.4 $[M+H]^+$, 1637.4 $[2M+H]^+$.

***N1,N1',N1'',N1'''*-(Benzene-1,2,4,5-tetrayl)tetrakis(N8-hydroxyoctanediamide). (MuH2344)**

Following general procedure B with 10 equivalents of $NH_2OH$ by amine, from compound **4** (160.0 mg, 0.20 mmol) with a stirring time increased to 3.5 h. The solid was dried to afford **MuH2344** as an orange powder (91.5 mg, 0.11 mmol, y. 57%). **$^1$H NMR** (600 MHz, DMSO): δ (ppm) = 10.34 (br, 4H, OH), 9.28 (br, 4H, NH), 8.67 (br, 4H, NH-OH), 7.65 (s, 2H, H-3'/6'), 2.30 (t, J = 7.4 Hz, 8H, H-7), 1.94 (t, J = 7.3 Hz, 7H, H-2), 1.57 (p, J = 7.7 Hz, 9H, H-6), 1.49 (p, J = 7.4 Hz, 8H, H-3), 1.28 (dt, J = 15.1, 7.8 Hz, 16H, H-4/5). **$^{13}$C NMR** (151 MHz, DMSO): δ (ppm) = 171.9 (C-8), 169.6 (C-1), 127.8 (C-1'/2'/4'/5'), 121.1 (C-3'/6'), 36.5 (C-7), 32.7 (C-2), 28.9 (C-6/3), 25.5 (C-4/5). **HRMS** Calcd for $C_{38}H_{63}N_8O_{12}$ 823.4560, Found 823.4557.

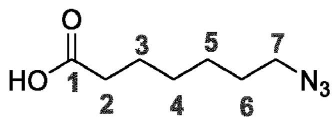

**7-Azidoheptanoic acid (5)**

7-bromoheptanoic acid (498.9 mg, 3.4 mmol, 1 eq.) was solubilized in DMF (6 ml). After addition of sodium azide (187.7 mg, 2.9 mmol, 1.2 eq.), the reaction mixture was stirred at reflux overnight. After completion of the reaction, $H_2O$ was added to the mixture and was extracted by EtOAc three times. The organic phase was washed three times with brine, dried over $Na_2SO_4$, filtered and concentrated *in vacuum.* **5** was obtained as a colorless oil and was used in the next step without further purification (360.3 mg, 2.1 mmol, y. 62%). **$^1$H NMR** (400 MHz, $CDCl_3$): δ (ppm) = 3.26 (t, J = 6.9 Hz, 2H, H-2), 2.36 (t, J = 7.4 Hz, 2H, H-7), 1.76 - 1.53 (m, 4H, H-3/6), 1.46 - 1.30 (m, 4H, H-4/5). **$^{13}$C NMR** (101 MHz, $CDCl_3$) δ (ppm) = 179.8 (C-1), 51.3 (C-7), 33.9 (C-2), 28.6 (C-3), 28.5, 26.3 (C-4/5), 24.4 (C-6).

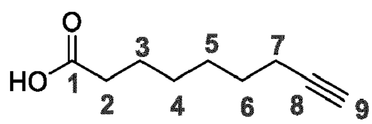

**Non-8-ynoic acid (6)**

Jone's reagent was prepared as followed: $CrO_3$ (312.4 mg, 3.2 mmol, 1.5 eq.) was solubilized in $H_2O$ (3.6 ml). The solution was colled down in an iced bath and sulfuric acid (0.23 ml, 2 eq.) was added dropwise, before addition of acetone (10 ml). Then, non-8-yne-1-ol (302.5 mg, 2.1 mmol, 1 eq.) was solubilized in 20 ml of acetone and added dropwise to Jone's reagent solution. The reaction mixture was stirred at 0 °C to room temperature until completion of the reaction. After 3 h, the reaction mixture was quenched with isopropanol, $H_2O$ was then added and extracted three times with EtOAc. The organic phases were washed with brine, dried over $Na_2SO_4$, filtered and concentrated *in vacuum.* The compound was purified by flash chromatography using a linear gradient ($CH_2Cl_2$/MeOH; 100/0 to 95/5) to afford **6** as a colorless oil (217.7 mg, 1.41 mmol, y. 65%). **R*f*=** 0.52 ($CH_2Cl_2$/MeOH 95/5). **$^1$H NMR** (400 MHz, $CDCl_3$) δ (ppm) = 2.34 (t, J = 7.5 Hz, 2H, H-2), 2.17 (td, J = 7.0, 2.6 Hz, 2H, H-7), 1.93 (t, J = 2.6 Hz, 1H, H-9), 1.63 (p, J = 7.4 Hz, 2H, H-3), 1.52 (p, J = 7.1 Hz, 2H, H-6), 1.45 - 1.27 (m, 4H, H-4/5). **$^{13}$C NMR** (101 MHz, $CDCl_3$) δ (ppm) = 180.5 (C-1), 84.6 (C-8), 68.4 (C-9), 34.1 (C-2), 28.6, 28.4 (C-4/5), 28.3 (C-6), 24.6 (C-3), 18.4 (C-7).

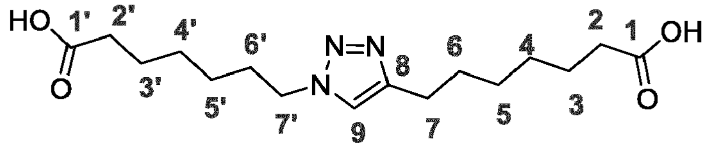

**7,7'-(1H-1,2,3-Triazole-1,4-diyl)diheptanoic acid.** (7)

Solutions of NaAsc 0.1 M in $H_2O$ and $Cu(OAc)_2$ 0.04 M in $H_2O$ were prepared. **6** (100.9 mg, 0.6 mmol, 1 eq.) and **5** (102.9 mg, 0.6 mmol, 1 eq.) were solubilized in tBuOH (3 ml). Previously prepared solutions of $Cu(OAc)_{2(aq)}$ (1.5 ml) and $NaAsc_{(aq)}$ (1.5 ml) were added and the mixture was stirred at room temperature. After completion of the reaction, monitored by HPLC (2 h), the mixture was diluted in $H_2O$ and extracted three times with $CH_2Cl_2$. The aqueous phases were acidified to pH=1 with HCl 1N and extracted again three times with $CH_2Cl_2$ and this was repeated twice. All organic phases were combined, dried over $Na_2SO_4$, filtered, and concentrated *in vacuum.* Compound **7** was obtained as a greenish powder (184.0 mg, 0.57 mmol, y. 94%). **$^1$H NMR** (400 MHz, $CDCl_3$): δ (ppm) = 7.31 (s, J = 2.3 Hz, 1H, H-9), 4.35 (t, J = 7.1 Hz, 2H, H-7'), 2.75 (t, J = 7.6 Hz, 2H, H-7), 2.37 (t, J = 7.3, 4H, H-272), 1.93 (t, J = 7.2 Hz, 2H, H-6'), 1.67 (m, 6H, H-6/3/3'), 1.40 (m, 8H, H-47574/5). **$^{13}$C NMR** (101 MHz, $CDCl_3$) δ (ppm) = 179.3, 178.9 (C-1/1'), 148.2 (C-8), 120.8 (C-9), 50.2 (C-2'), 34.2, 34.0 (C-2'/2), 30.1 (C-6'), 29.2 (C-6), 28.8, 28.4, 26.2 (C-4/5/4'/5'), 25.4 (C-7), 24.7, 24.5 (C-3/3'). **ESI-MS** *m*/*z*=326.4 $[M+H]^+$, 651.5 $[2M+H]^+$.

**7,7'-(1H-1,2,3-Triazole-1,4-diyl)bis(N-(trityloxy)heptanamide). (8)**

Compound **7** (44.6 mg, 0.14 mmol, 1 eq.) was solubilized in DMF (2.8 ml). After addition of HATU (114.65 mg, 0.3 mmol, 2.2 eq.), $NH_2OTrt$ (85.2 mg, 0.3 mmol, 2.2 eq.) and DIPEA dropwise (71 μL, 0.41 mmol, 3 eq.), the reaction mixture was stirred at room temperature overnight. After completion of the reaction monitored by HPLC, the mixture was diluted with $H_2O$ and extracted three times with $CH_2Cl_2$. The organic phases were washed with brine, dried over $Na_2SO_4$, filtered and concentrated *in vacuum.* The crude mixture was purified by preparative HPLC using a linear gradient ($H_2O/CH_3CN$: 60/40 to 0/100) to afford **8** as a white powder (73.3 mg, 0.09 mmol, y. 64%). **$^1$H NMR** (400 MHz, $CDCl_3$) δ (ppm) = 7.33 (3, 15H, H-Trt), 7.19 (s, 1H, H-9), 4.24 (t, J = 7.2 Hz, 2H, H-7'), 2.65 (t, J = 7.7 Hz, 2H, H-7), 1.80 (q, J = 7.4 Hz, 2H, H-6'), 1.57 (d, J = 8.5 Hz, 6H, H-6/3/3'), 1.32 - 0.95 (m, 12H, H-4/5/4'/5'). **ESI-MS** *m*/*z*=840.6 $[M+H]^+$, 1680.3 $[2M+H]^+$.

**7,7'-(1H-1,2,3-Triazole-1,4-diyl)bis(N-hydroxyheptanamide). (MuH2321)**

Compound **8** (73.3 mg, 0.09 mmol, 1 eq.) was solubilized in 2 ml of $CH_2Cl_2$, the resulting solution was cooled down in an iced bath. Then, 20 μl of Tis and 0.1 ml of TFA were added successively. The reaction mixture color turned to yellow, and it was stirred at 0°C for 30 min. The crude mixture was precipitated in ether and centrifugated (15 min, 4500 rpm) twice to remove all trityl salts. **MuH2321** was obtained as a pinkish powder (26.7 mg, 0.07 mmol, y. 86%). **$^1$H NMR** (600 MHz, DMSO): δ (ppm) = 10.32 (br, 2H, NH), 7.82 (s, 1H, H-9), 4.26 (t, J = 7.1 Hz, 2H, H-7'), 2.58 (t, J = 7.6 Hz, 2H, H-7), 1.92 (2 t, J = 7.2 Hz, 4H, H-2/2'), 1.76 (p, J = 7.2 Hz, 2H, H-6'), 1.56 (p, J = 7.5 Hz, 2H, H-6), 1.46 (m, 4H, H-3/3'), 1.33 - 1.14 (m, 8H, H-4/5/475'). **$^{13}$C NMR** (151 MHz, DMSO): δ (ppm) = 169.5 (C-1/1'), 147.2 (C-8), 122.0 (C-9), 49.5 (C-7'), 32.7 32.6 (C-2/2'), 30.1 (C-6'), 28.8 (C-6), 28.4 26.1 (C-4/5/4'/5'), 25.5 (C-3/3'), 25.4 (C-7). **HRMS** Calcd for $C_{16}H_{30}N_5O_4$ 356.2292, Found 356.2293.

Example 2: Synthesis RAFT derivatives

The inventors prepared platforms or RAFT, which corresponds to a cyclic peptide with a sequence as set forth in SEQ ID NO: 5 and SEQ ID NO: 10.

Synthesis scheme is shown in **Figure 1.**

Peptides are then modified in order to modify lysine (K) residue in order to introduce azide group. Synthesis scheme is shown in **Figure 2.**

The azido cyclic peptides, illustrated in Figure 2 by SEQ ID NO: 41 are ready to be linked to HDAC inhibitors.

**Resin functionalisation**

2-chlorotrityl chloride resin (3.0 g, 4.8 mmol, 1 eq.) was suspended in 30 ml ($DMF/CH_2Cl_2$: 5/5), then Fmoc-Gly-OH (4.31 g, 14,4 mmol, 3 eq.) and DIPEA (4 ml, 24 mmol, 5 eq.) were added. After two days stirring at room temperature, the resin was filtered and neutralized during 1 h in 30 ml methanol. Then the resin was filtered, washed with $CH_2Cl_2$ three times, DMF twice and diethyl ether once, and dried *in vacuo.* In order to determine the resin loading, 10 mg of resin was

suspended in 1 ml of a solution of piperidine/DMF: 2/8 for 30 min. Then, 100 μl of the solution was suspended in 9.9 ml of DMF and UV-Vis absorbance was measured at 301 nm. The resin charge was determined as:

[Math 1]

$$loading = \frac{Abs \times V_{tot}}{\varepsilon \times l},$$

where *Abs* is the absorbance measured, $V_{tot}$ is the volume of the overall solution prepared (μl), $\varepsilon$ is the molar attenuation coefficient ($M^{-1}.cm^{-1}$) (7800 $M^{-1}.cm^{-1}$ at 301 nm for the dibenzofulvene-piperidine) and *l* represents the length of the cuvette (cm).

In fine, 4.33 g of resin with a charge of 0.55 mmol/g was obtained.

**RAFT-4K**

Linear peptide: SPPS in Fmoc strategy. The synthesis of $H_2N$-K(Boc)-A-K(Boc)-P-G-K(Boc)-A-K(Boc)-P-G-H sequence was performed manually in a reactor. Solutions of each compound was prepared previously as: HATU 0.2 M in DMF, aa 0.6 M in DMF and piperidine/DMF: 2/8. The glycine functionalized 2-chlorotrytil resin (672 mg, 0.37 mmol, 1 eq.) was first wet with DMF. Each cycle deprotection of the Fmoc and coupling was performed for each amino acid as: deprotection twice with 10 ml of piperidine/DMF during 1 min, resin washed with DMF, coupling twice during 10 min with a solution of 3 ml of Fmoc-aa-OH (1.85 mmol, 5 eq.), 9.20 ml of HATU (1.85 mmol, 5 eq.), and 0.63 ml of DIEPA (3.7 mmol, 10eq.), resin washed with DMF. After introduction of the 9 amino acids, the last Fmoc group was deprotected as before. Then, the peptide was cleaved from the resin by 10 ml of 1% TFA in $CH_2Cl_2$ four times. TFA was neutralized with 20 ml of MeOH/pyridine: 8/2. The medium was concentrated *in vacuum,* precipitated in diethyl ether and centrifuged (15 min, 4500 rpm). Pellets was solubilized in $H_2O/CH_3CN$ and purified by preparative HPLC using a linear gradient ($H_2O/CH_3CN$ + 0.1% TFA; 85/15 to 50/50) to afford the linear RAFT-4K(Boc) (407 mg, 0.30 mmol, y. 80%) as a white powder. **ESI-MS** m/z=1382.3 $[M+H]^+$, 691.6 $[M+2H]^+$.

Cyclisation/deprotection: the linear peptide was solubilized in DMF (640 ml, 0.5 nM) with PyBop (180 mg, 0.48 mmol, 1.5 eq.) and DIPEA (0.61 ml, 3.52 mmol, 11 eq.). The reaction mixture was stirred at room temperature for 2 h. After completion of the reaction (monitored by HPLC), the medium was concentrated *in vacuum.* The residue was then solubilized in 30 ml TFA/$H_2O$/Tis: 95/2.5/2.5 and stirred at 40 °C for 1 h 30. After completion of the reaction, the medium was concentrated *in vacuum,* precipitated in ether and centrifuged (15 min, 4500 rpm). Pellets were solubilized in $H_2O$ and purified by preparative HPLC using a linear gradient ($H_2O/CH_3CN$ + 0.1% TFA; 100/0 to 80/20) to afford RAFT-4K as a white powder (114 mg, 0.12 mmol, y. 40%). **ESI-MS** m/z=963.3 $[M+H]^+$, 482.5 $[M+2H]^+$, 1075.4 $[M+TFA-H]^-$.

**RAFT-4K($N_3$)**

Linear peptide: $H_2N$-K($N_3$)-A-K($N_3$)-P-G-K($N_3$)-A-K($N_3$)-P-G-H was synthetized following the same procedure as the RAFT-4K except some modifications. The coupling of Fmoc-Lys($N_3$)-OH was performed during 20 min with a solution of 1.85 ml Fmoc-Lys($N_3$)-OH (1.11 mmol, 3 eq.), 5.55ml of HATU (1.11 mmol, 3 eq.) and 378 μl DIPEA (2.22 mmol, 6 eq.). The cleavage of the resin was performed with 10 ml of a solution of 95% TFA in $CH_2Cl_2$. The compound was purified by preparative HPLC using a linear gradient ($H_2O/CH_3CN$ + 0.1% TFA; 80/20 to 50/50) to afford linear RAFT-4K($N_3$) as a white powder (217 mg, 0.2 mmol, y. 54%). **ESI-MS** m/z=1086.1 $[M+H]^+$, 543.4 $[M+2H]^+$.

Cyclisation: the cyclisation was performed using the same procedure as RAFT-4K. The compound was purified by preparative HPLC using a linear gradient ($H_2O/CH_3CN$ + 0.1% TFA; 80/20 to 50/50) to afford RAFT-4K($N_3$) as a white powder (123 mg, 0.12 mmol, y. 60%). **ESI-MS** m/z=1067.7 $[M+H]^+$, 1089.3 $[M+Na]^+$, 1065.3 $[M-H]^-$.

**RAFT-2K**

Linear peptide: $H_2N$-A-A-K(Boc)-P-G-A-A-K(Boc)-P-G-H was synthetized following the same procedure as the RAFT-4K except some modifications. The coupling of the second glycine residue was performed three times instead of two. The compound was purified by preparative HPLC using a linear gradient ($H_2O/CH_3CN$ + 0.1% TFA; 95/05 to 50/50) to afford linear protected RAFT-2K as a white powder (137.6 mg, 0.13 mmol, y. 31%). **ESI-MS** m/z=1067.6 $[M+H]^+$, 534.4 $[M+2H]^+$.

Cyclisation/deprotection: the cyclisation was performed using the same procedure as RAFT-4K for 2 h. Then, cyclic peptide was deprotected using the procedure of RAFT-4K for 2 h. The compound was purified by preparative HPLC using a linear gradient (H2O/$CH_3CN$ + 0.1 % TFA; 100/0 to 70/30) to afford **RAFT-2K** as a white powder (78.7 mg, 0.093 mmol, y. 71%). **ESI-MS** *m/z*=849.5 $[M+H]^+$, 425.4 $[M+2H]^+$.

Example 3: Synthesis of hydoxamic acid-based multivalent compounds involving a peptide platform

3.1 - Dimers

*Compounds; wherein RAFT-2K is SEQ ID NO: 5

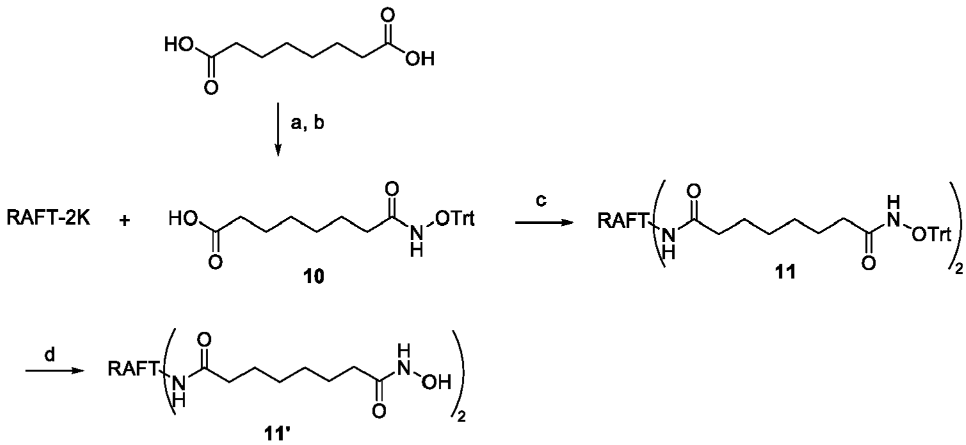


10 11 11'

**(a)** Anhydride acetic, reflux, 3.5 h; **(b)** $NH_2OTrt$, THF, Ar, 2 d.; **(c)** HATU, DIPEA, DMF, RT, 2 d.; **(d)** TFA, $CH_2Cl_2$, Tis, 0°C to RT, 1 h.

*Compound MuH wherein RAFT-2K($N_3$) is SEQ ID NO: 41

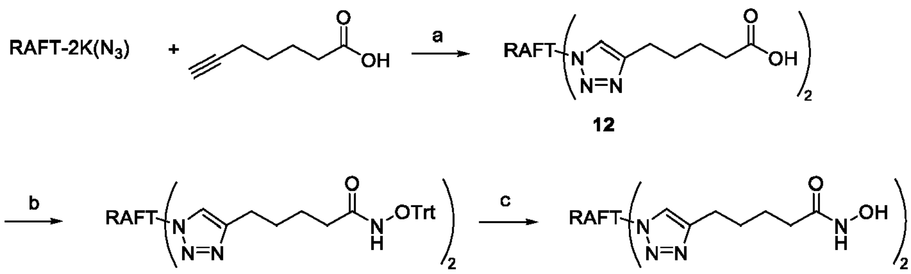


12 13 13'

**(a)** $Cu(OAc)_2$, NaAsc, $tBuOH/H_2O$, RT, 5 h; **(b)** $NH_2OTrt$, EDCI.HCl, HOBt, NMM, Ar. 1 d. ; **(c)** TFA, $CH_2Cl_2$, Tis, RT, 30 min.

*Compound MuH2357; wherein RAFT-2K(phenylamine)

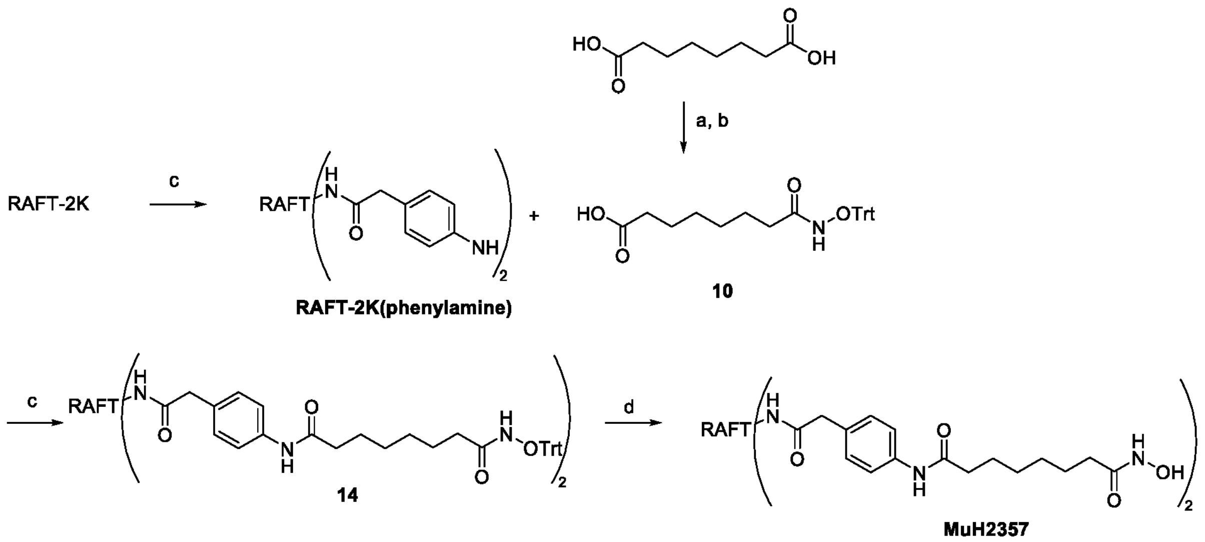

RAFT-2K(phenylamine)
10
14
MuH2357

**(a)** Anhydride acetic, reflux, 3.5 h; **(b)** $NH_2OTrt$, THF, Ar, 2 d.; **(c)** HATU, DIPEA, DMF, RT, 2 d.; **(d)** TFA, $CH_2Cl_2$, Tis, 0°C to RT, 1 h.

3.2 - Tetramers

*Compounds MuH2120 and MuH2121; wherein RAFT-4K is SEQ ID NO: 10

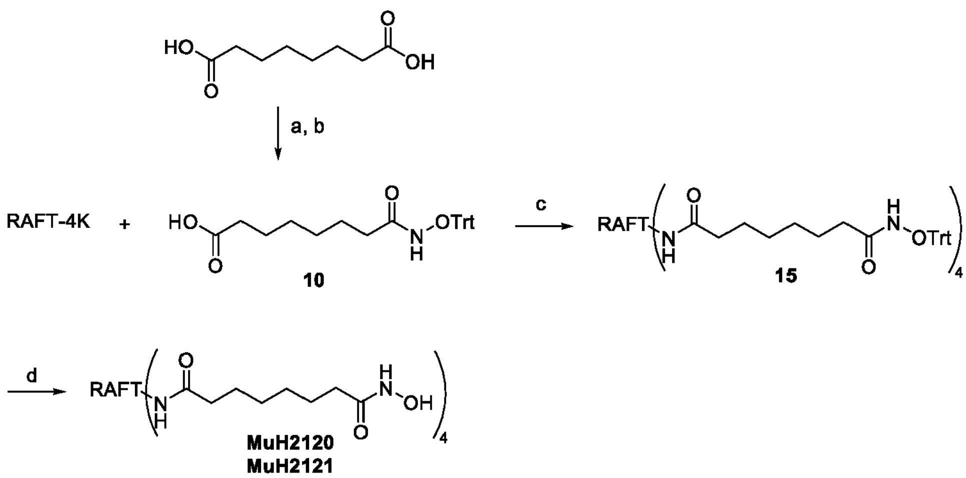

10
15
MuH2120
MuH2121

**(a)** Anhydride acetic, reflux, 3.5 h; **(b)** $NH_2OTrt$, THF, Ar, 2 d.; **(c)** HATU, DIPEA, DMF, RT, 2 d.; **(d)** TFA, $CH_2Cl_2$, Tis, RT, 30 min.

*Compound MuH2218; wherein RAFT-4K ($N_3$) is SEQ ID NO: 41

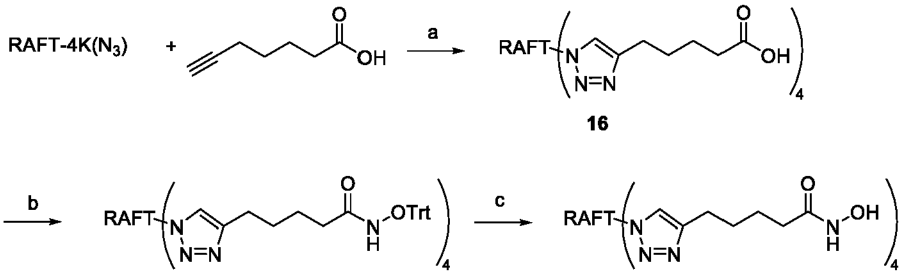


16

17

MuH2218

**(a)** $Cu(OAc)_2$, NaAsc, $tBuOH/H_2O$, RT, 5 h; **(b)** $NH_2OTrt$, EDCI.HCl, HOBt, NMM, Ar. 1 d..; **(c)** TFA, $CH_2Cl_2$, Tis, RT, 30 min.

*Compound MuH wherein RAFT-4K(phenylamine)

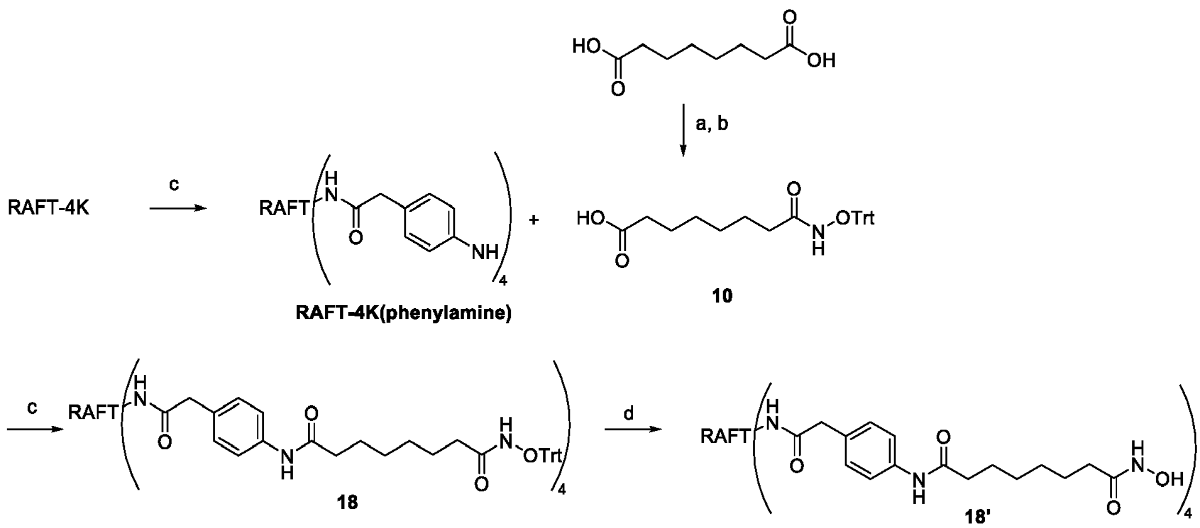


RAFT-4K(phenylamine)

10

18

18'

(a) Anhydride acetic, reflux, 3.5 h; (b) $NH_2OTrt$, THF, Ar, 2 d.; (c) HATU, DIPEA, DMF, RT, 2 d.; **(d)** TFA, $CH_2Cl_2$, Tis, 0°C to RT, 1 h.

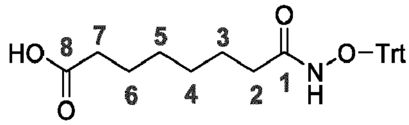

**8-oxo-8-((trityloxy)amino)octanoic acid. (10)**

Suberic acid (3 g, 17.2 mmol, 1 eq.) was solubilized in anhydride acetic (6.5 g, 6 ml, 63.7 mmol, 3.7 eq.) and heated at reflux until completion of the reaction (3.5 h). The medium was concentrated under vacuum until dryness. Then, $NH_2OTrt$ (4.8 g, 17.4 mmol, 1.01 eq) was added and the flask was degassed with Argon. Powders were solubilized in anhydrous THF (40 ml) and the medium was stirred during 2 days under Argon atmosphere. The reaction mixture was quenched with saturated $NaHCO_3$ solution and extracted with EtOAC. The aqueous phase was acidified to pH = 2 and extracted again with EtOAc. The organic phases were added, dried over $Na_2SO_4$, filtered, and concentrated under vacuum. The compound was purified by manual flash chromatography ($CH_2Cl_2$/MeOH + 5% acetic acid: 100/0 to 50/50) to afford **10** as a white powder (662 mg, 1.53 mmol, y. 9 %). **R*f***= 0.33 ($CH_2Cl_2$/MeOH 95/5). **$^1$H NMR** (400 MHz, $CDCl_3$): δ (ppm) = 7.38 (s, 15H, H-Trt), 2.29 (t, J = 7.5 Hz, 2H, H-2), 1.64 (t, J = 7.4 Hz, 2H, H-7), 1.55 (p, J = 7.5 Hz, 2H, H-3), 1.34 - 0.99 (m, 6H, H-4/5/6). **$^{13}$C NMR** (101 MHz, $CDCl_3$): δ (ppm) = 179.0 (C-1), 177.7 (C-8), 141.1 (C-Trt), 134.2 - 117.3 (CH-Trt), 93.4 ($C_{IV}$-Trt), 33.8 (C-2), 31.1 (C-7), 28.7 (C-6), 24.5 (C-3), 23.3 (C-4/5). **ESI-MS** *m*/*z*=430.1 $[M-H]^-$ , 861.2 $[2M-H]^-$.

**General procedure for RAFT-( 8-oxo-8-((trityloxy)amino)octanoic acid)$_2$ (11) and RAFT-( 8-oxo-8-((trityloxy) amino)octanoic acid)$_4$ (15).**

RAFT-4K (10.0 mg, 10.3 μmol, 1 eq.), compound **19** (44.4 mg, 0.1 mmol, 2.5 eq; by amine), HATU (39.1 mg, 0.1 mmol, 2.5 eq. by amine) were solubilized in DMF (640 μl). Then, DIPEA (45 μl, 5 eq. by amine) was added, and the reaction mixture was stirred at room temperature 2 days. After completion of the reaction, the solvent was evaporated *in vacuum.* The crude product was used in the next step without further purification. **ESI-MS** *m*/*z*=1309.2 $[M+H]^+$, 1307.0 $[M-H]^-$.

**General procedure for RAFT-(methyl 8-(hydroxyamino)-8-oxooctanoate)$_2$ and RAFT-(methyl 8-(hydroxyamino)-8-oxooctanoate)$_2$ and RAFT-(methyl 8-(hydroxyamino)-8-oxooctanoate)$_2$ (11') and RAFT-(methyl 8-(hydroxyamino)-8-oxooctanoate)$_4$. (MuH2120/MuH2121)**

The crude and protected hydroxamic multivalent compound was solubilized in 5.3 ml TFA/MeOH/$CH_2Cl_2$/Tis (55/35/2/8) and stirred at room temperature 2 h. The mixture was concentrated *in vacuum* and purified by semi-preparative HPLC using a linear gradient ($H_2O/CH_3CN$ + 0.1 % TFA; 95/5 to 0/100)

For the tetravalent compound, two different fractions were afforded, corresponding to **MuH2120** as a white powder (2.0 mg, 1.2 μmol, y. 12 %). **ESI-MS** m/z=1647.6 $[M+H]^+$, 824.6 $[M+2H]^{2+}$, and **MuH2121** as a white powder (2.3 mg, 1.4 μmol, y. 14 %). **ESI-MS** *m*/*z*=1647.7 $[M+H]^+$, 824.2 $[M+2H]^{2+}$.

**General procedure for RAFT-(5-(1-X-1H-1,2,3-triazol-4-yl)pentanoic acid)$_2$ (12) and RAFT-(5-(1-X-1H-1,2,3-triazol-4-yl)pentanoic acid)$_4$. (16)**

Solutions of NaAsc 0.09 M in $H_2O$ and $Cu(OAc)_2$ 0.09 M in $H_2O$ were first prepared. RAFT-4K($N_3$) (18.82 mg, 0.017 mmol, 1 eq.) and 6-heptynoic acid (13.35 mg, 14 μL, 0.11 mmol, 1.5 eq. by amine) were solubilized in tBuOH (300 μL). $Cu(OAc)_{2(aq)}$ (200 μL, 3.2 mg, 0.017 mmol, 0.25 eq. by amine) and $NaAsc_{(aq)}$ (150 μL, 2.6 mg, 0.013 mmol, 0.18 eq. by amine) were added and the mixture was stirred at room temperature. After completion of the reaction, monitored by HPLC (5 h), the mixture was freeze-dried and purified by semi-preparative HPLC using a linear gradient (H2O/$CH_3CN$ + 0.1 % TFA; 90/10 to 30/70) to afford **22** as a white powder (22.9 mg, 0.015 mmol, y. 88%). **ESI-MS** *m*/*z*=1571.6 $[M+H]^+$, 786.9 $[M+2H]^{2+}$.

**General procedure for RAFT-(5-(1-X-1H-1,2,3-triazol-4-yl)-N-(trityloxy)pentanamide)$_2$ (13) and RAFT-(5-(1-X-1H-1,2,3-triazol-4-yl)-N-(trityloxy)pentanamide)$_4$ (17)**

Compound **16** (22.9 mg, 0.015 mmol, 1 eq.), HOBt (9.52 mg, 0.06 mmol, 1.1 eq. by amine) and 380 μl of DMF were flushed with Argon. Then EDCI.HCl (16.8 mg, 0.09 mmol, 1.5 eq. by amine) in 119 μl of DMF and *N*-methylmorpholine (19 μL, 3 eq. by amine) were added to the mixture followed by the addition of $NH_2OTrt$ (32.1 mg, 0.12 mmol, 2 eq. by amine) in 120 μl of DMF. The reaction mixture was stirred at room temperature, under Argon atmosphere for 1 day. Then, the mixture was solubilized un $CH_2Cl_2$, washed with saturated NaHCOs solution and brine. The organic phase was dried over $Na_2SO_4$, filtered and concentrated. The crude was purified by semi-preparative HPLC using a linear gradient ($H_2O/CH_3CN$ + 0.1 % TFA; 80/20 to 0/100) to afford **17** protected as a white powder (19.9 mg, 7.6 μmol, y. 51%). **ESI-MS** m/z=1301.3 $[M+2H]^{2+}$, 868.0 $[M+3H]^{3+}$.

**General procedure for RAFT-(*N*-hydroxy-5-(1-methyl-1H-1,2,3-triazol-4-yl) pentanamide)$_4$ (13') and RAFT-(*N*-hydroxy-5-(1-methyl-1H-1,2,3-triazol-4-yl)pentanamide)$_4$. (MuH2218)**

**17** was solubilized in 1 ml $CH_2Cl_2$/TFA/Tis (95/2.5/2.5) and stirred at room temperature upon completion of the reaction, monitored by HPLC. After 1 h, the mixture was precipitated in ether and centrifugated. The pellet was purified by semi-preparative HPLC using a linear gradient (H2O/$CH_3CN$ + 0.1 % TFA; 95/05 to 50/50) to afford **MuH2218** as a white powder (4.0 mg, 2.5 μmol, y. 33%). **ESI-MS** *m*/*z*=1632.5 $[M+H]^+$, 816.9 $[M+2H]^{2+}$, 544.9 $[M+3H]^{3+}$.

**RAFT-2K(phenylamine)**

**2-(4-(tert-butoxycarbonyl)amino)phenyl)acetic acid** (89.4 mg, 0.35 mmol, 10 eq.), and HATU (143.2 mg, 0.35 mmol, 10 eq.)were solubilized in dry DMF (1.2 ml), followed by addition of 60 μl of DIEA. The reaction mixture was stirred 15 min at RTand **RAFT-2K** (30.0 mg, 32 μmol, 1 eq.) was added, followed by 60 μl of DIEA. The mixture was stirred overnight at RT until completion of the reaction monitored by HPLC. The crude mixture was directly purified by preparative HPLC using a linear gradient ($H_2O/CH_3CN$ + 0.1% TFA; 85/15 to 0/100) to afford the protected RAFT-2K(phenylamine-boc) as a white powder (50 mg, 35 μmol, quant.). **ESI-MS** *m*/*z*=1315.5 $[M+H]^+$.

The protected functionalized peptide was then deprotected in 10 ml of TFA/$H_2O$/Tis (95/2.5/2.5). The reaction mixture was stirred at RT 1 h and then concentrated. The crude mixture was purified by semi-preparative HPLC using a linear gradient ($H_2O/CH_3CN$ + 0.1% TFA; 95/5 to 50/50) to afford RAFT-2K(phenylamine) as a white powder (16.3 mg, 14.6 μmol, y. 40%). **ESI-MS** *m*/*z*=1115.9 $[M+H]^+$.

**RAFT-4K(phenylamine)**

**2-(4-(tert-Butoxycarbonyl)amino)phenyl)acetic acid** (66.4 mg, 0.26 mmol, 10 eq.), and HATU (98.6 mg, 0.26 mmol, 10 eq.) were solubilized in dry DMF (0.9 ml), followed by addition of 90 μl of DIEA. The reaction mixture was stirred 15 min at RT and **RAFT-4K** (25.0 mg, 26 μmol, 1 eq.) was added. The mixture was stirred 2 h at RT until completion of the reaction followed by HPLC. The crude mixture was directly purified by preparative HPLC using a linear gradient ($H_2O/CH_3CN$ + 0.1% TFA; 70/30 to 0/100) to afford the protected RAFT-4K(phenylamine-boc) as a white powder (21.1 mg, 11.1 μmol, y. 32 %). **ESI-MS** *m*/*z*=1895.8 $[M+H]^+$.

The protected functionalized peptide was then deprotected in 5 ml of TFA/$H_2O$/Tis (95/2.5/2.5). The reaction mixture was stirred at RT 30 min and then concentrated. The crude mixture was precipitated in ether and centrifugated (4500 rpm, 15 min, 20 °C). RAFT-4K(phenylamine) was obtained as a white solid (20.4 mg, 13.6 μmol, quant.). **ESI-MS** *m*/*z*=1496.35 $[M+H]^+$.

**General Procedure for RAFT-( $N^1$-(4-(2-amino-2-oxoethyl)phenyl)-$N^8$-(trityloxy)octane diamide)$_2$ (14) and RAFT-($N^1$-(4-(2-amino-2-oxoethyl)phenyl)-$N^8$-(trityloxy)octane diamide))$_4$. (18)**

Compound **10** (10.7 mg, 48 μmol, 2.5 eq. by amine) and HATU (21.7 mg, 48 μmol, 2.5 eq. by amine) were solubilized in 340 μl of dry DMF, followed by addition of 17 μl of DIEA (5 eq. by amine). The mixture was stirred 10 min at RT and **RAFT-2K(phenylamine)** (10.7 mg, 9.6 μmol, 1 eq.) was added. The mixture was stirred at RT 1 h, until completion of the reaction monitored by HPLC. The crude mixture was directly purified by semi-preparative HPLC using a linear gradient ($H_2O/CH_3CN$ + 0.1% TFA; 70/30 to 0/100) to afford **14** as a white powder (11.0 mg, 5.7 μmol, y. 59%). **ESI-MS** *m*/*z*=1941.6 $[M+H]^+$.

**General Procedure for RAFT-($N^1$-(4-(2-amino-2-oxoethyl)phenyl)-$N^8$-hydroxyoctane diamide))$_2$ (MuH2357) and RAFT-($N^1$-(4-(2-amino-2-oxoethyl)phenyl)-$N^8$-hydroxyoctanediamide))$_4$. (14')**

Compound **14** (11.0 mg, 5.7 μmol, y. 59%), was solubilized at 0°C in 0.5 ml of MeOH. Then, 60 μl of Tis and 60 μl of TFA were added to the mixture, that was stirred at 0°C to room temperature for 1 h. The crude mixture was precipitated in ether and centrifugated twice (4500 rpm, 15 min, RT) to remove all trityl salts. **MuH2357** was obtained as a white powder (3.4 mg, 2.3 μmol, 40 %). **HRMS** Calcd for $C_{70}H_{105}N_{16}O_8$ 1457.7787, Found 1457.7787.

Example 4: Synthesis of functionalised benzamides

In order to produce the compounds according to the invention, the inventors prepared functionalised benzamide derivatives according to the following reactions:

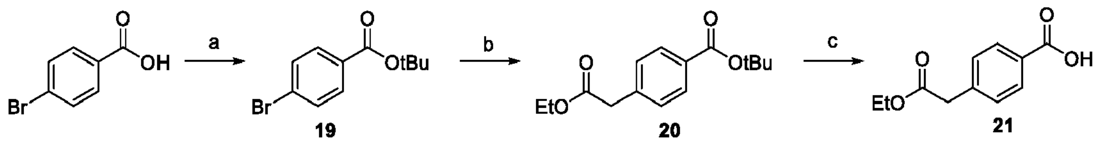

19 20 21

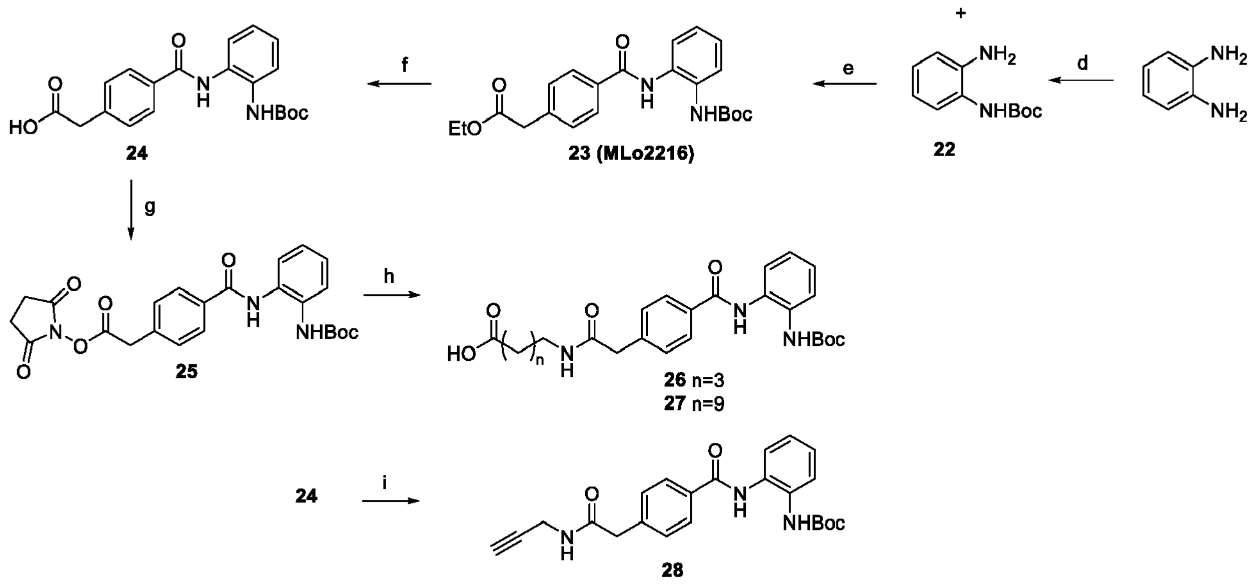


24 23 (MLo2216) 22 25 26 n=3 27 n=9 24 28

**(a)** $Boc_2O$, DMAP, dry THF, reflux, 1 d; **(b)** diethyl malonate, $KHCO_3$, $K_2CO_3$, $Pd(tbu)_2$, 160 °C, Ar, 8 h; **(c)** TFA 30%, $CH_2Cl_2$, RT, 30 min; **(d)** $Boc_2O$, DMF, 40 °C, 180 mbar, 10 min; **(e)** HATU, DIPEA, DMF, 0 °C to RT, 18 h; **(f)** NaOH 1M, THF/$H_2O$, RT, 16 h; **(g)** NHS, DCC, $CH_3CN$, RT, 6 h ; **(h)** various amino acid alkyl chain, DMF, RT, 8 to 16 h; **(i)** propargylamine, DCC, DMAP, $CH_2Cl_2$, RT, 5 h.

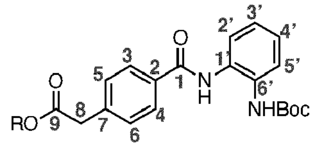

***tert*-Butyl 4-bromobenzoate (19).**

4-Bromobenzoic acid (2.0 g, 9.95 mmol, 1 eq.), $Boc_2O$ (2.6 g, 11.9 mmol, 1.2 eq.) and DMAP (0.61 mg, 5.0 mmol, 0.5 eq.) were dissolved in anhydrous THF (15 ml). The solution was heated to reflux and stirred overnight under argon. Then, the medium was concentrated to dryness. The residue was dissolved in ethyl acetate and washed with saturated $NaHCO_3$ solution and brine. The organic layer was dried over anhydrous $Na_2SO_4$, filtered, and concentrated under *vacuum.* to afford **19** as a yellow oil (1.48 g, 5.76 mmol, y. 57%). **R*f*=** 0.73 (cyclohexane/EtOAc 9/1). **$^1$H NMR** (400 MHz, $CDCl_3$): δ (ppm) = 7.89 - 7.80 (td, *J* = 2.4, 8.4 Hz, 2H, H-3/4), 7.59 - 7.50 (td, *J* = 2.4, 8.4 Hz, 2H, H-5/6), 1.59 (s, 9H, H-tBu). **$^{13}$C NMR** (101 MHz, $CDCl_3$): δ (ppm) = 165.1 (C-1), 131.6 (C-3/4), 131.1 (C-5/6), 131.0 (C-7), 127.5 (C-2), 81.6 ($C_{IV}$tBu), 28.3 (**C**-tBu). **ESI-MS** *m*/*z*=1067.7 $[M+H]^+$, 1089.3 $[M+Na]^+$, 1065.3 $[M-H]^-$.

***tert*-Butyl 4-(2-ethoxy-2-oxoethyl)benzoate (20).**

Tert-butyl 4-bromobenzoate **19** (885 mg, 3.44 mmol, 1 eq.), potassium carbonate (712 mg, 0.34 mmol, 1.5 eq.), potassium bicarbonate (520 mg, 5.16 mmol, 1.5 eq.) and bis(tri-*tert*-butylphosphine)palladium(0) (175 mg, 0.34 mmol, 0.1 eq) were placed in an oven-dried roundbottom flask equipped with a condenser. The flask was evacuated and filled with argon three times. Then, the medium was solubilized in diethyl malonate (3.45 ml, 22.72 mmol, 6.6 eq.) stirred and heated at 160 °C under argon for 8 h. After completion of the reaction, the medium was filtered through a pad of Celite and washed with ethyl acetate. The solution was washed with $H_2O$ and brine, and then dried over $Na_2SO_4$, filtered and concentrated under *vacuum.* The residue was purified by flash chromatography using a linear gradient (cyclohexane/EtOAc; 90/10 to 85/15) to afford **20** as a yellow oil (657 mg, 2.49 mmol, y. 72%). **R*f*=** 0.34 (cyclohexane/AcOEt 9/1).

**$^1$H NMR** (400 MHz, $CDCl_3$) δ (ppm): = 7.98 - 7.91 (td, *J* = 1.6, 8.8 Hz, 2H, H-3/4), 7.36 - 7.30 (td, *J* = 1.6, 8.8 Hz, 2H, H-5/6), 4.15 (q, *J* = 7.1 Hz, 2H, $CH_2$-OEt), 3.65 (s, 1H, H-8), 1.58 (s, J = 1.7 Hz, 9H, H-tBu), 1.24 (t, J = 7.2 Hz, 3H, $CH_3$-OEt). **$^{13}$C NMR** (101 MHz, $CDCl_3$): δ (ppm) = 171.0 (C-9), 165.7 (C-1), 138.9 (C-7), 131.1 (C-2), 129.8 (C-3/4), 129.3 (C-5/6), 81.1 ($C_{IV}$tBu), 61.2 ($CH_2$-OEt), 41.6 (C-8), 28.3 (C-tBu), 14.3 ($CH_3$-OEt).

**4-(2-Ethoxy-2-oxoethyl)benzoic acid (21).**

Compound **20** (545 mg, 2.06 mmol, 1 eq.) was solubilized in a solution of $CH_2Cl_2$/TFA: 7/3 and stirred at room temperature during 1 h. After completion of the reaction, the medium was concentrated *in vacuum* and dried. The residue was used in the next step without any purification. **R*f*=** 0.19 (cyclohexane/EtOAc 7/3).

**$^1$H NMR** (400 MHz, $CDCl_3$): δ (ppm) = 8.10 - 8.06 (td, *J* = 2.0, 9.2 Hz, 2H, H-3/4), 7.47 - 7.35 (td, *J* = 2.0, 9.2 Hz, 2H, H-5/6), 4.17 (q, *J* = 7.1 Hz, 1H, $CH_3$-OEt), 3.70 (s, 1H, H-8), 1.26 (t, *J* = 7.1 Hz, 2H, $CH_2$-OEt). **$^{13}$C NMR** (101 MHz, $CDCl_3$): δ (ppm) = 172.0 (C-1), 170.9 (C-9), 140.4 (C-7), 130.6 (C-3/4), 129.6 (C-5/6), 128.3 (C-2), 61.3 ($CH_2$-OEt), 41.6 (C-8), 14.3 ($CH_3$-OEt). **ESI-MS** *m*/*z*=207.0 $[M-H]^-$, 415.0 $[2M-H]^-$.

***tert*-Butyl (2-aminophenyl)carbamate (22).**

Benzene-1,2-diamine (1.02 g, 9.7 mmol, 1 eq.) and Boc anhydride (2.18 g, 10.0 mmol, 1.03 eq) were solubilized in DMF (10 ml). The reaction was carried out in a rotary evaporator at 40 °C and 180 mbar during 10 min. The reaction mixture was diluted in $CH_2Cl_2$ (30 ml) and washed with citric acid 5% (pH=3), $H_2O$ and brine. The organic layer was dried over $Na_2SO_4$, filtered and concentrated under vacuum. The residue was purified by flash chromatography using a linear gradient (cyclohexane/EtOAc; 100/0 to 50/50) to afford **22** as an amorphous solid (1.30 g, 6.24 mmol, y. 66%). **R*f*=** 0.78 (cyclohexane/EtOAc; 5/5).

**$^1$H NMR** (400 MHz, $CDCl_3$): δ (ppm) = 7.27 (d, *J* = 7.6 Hz, 1H, H-2'), 7.00 (td, *J* = 7.6, 1.5 Hz, 1H, H-3'), 6.80 (m, 2H, H-4'/5'), 6.24 (br, NH) 1.52 (s, 9H, H-Boc). **$^{13}$C NMR** (101 MHz, $CDCl_3$): δ (ppm) = 153.9 (C-Boc), 139.9 (C-1'), 126.2 (C-3'/5'), 124.9 (C-2'), 119.7 (C-6'), 117.7 (C-4'), 80.6 ($C_{IV}$-Boc), 28.4 ($CH_3$-Boc).

**Ethyl 2-(4-((2-((*tert*-butoxycarbonyl)amino)phenyl)carbamoyl)phenyl)acetate (23).**

Compound **21** (2.61 mmol, 1 eq.) was solubilized in DMF (25 ml). After addition of HATU (1.09 mg, 2.87 mmol, 1.1 eq.), tert-butyl (2-aminophenyl)carbamate (599.6 mg, 2.87 mmol, 1.1 eq.) and DIPEA dropwise (1.82 ml, 10.4 mmol, 4 eq.), reaction mixture was stirred at room temperature overnight. After completion of the reaction, the medium was concentrated *in vacuum.* The residue was solubilized in $H_2O$ and extracted with ethyl acetate three times. The organic layers were washed with brine, dried over $Na_2SO_4$, filtered and concentrated under *vacuum.* The compound was purified by manual flash chromatography (cyclohexane/EtOAc: 80/20 to 70/30) to afford **23** as a yellow oil (985.9 mg, 2.47 mmol, y. 89%). **R*f*=**0.45 (cyclohexane/EtOAc 6/4).

**$^1$H NMR** (400 MHz, $CDCl_3$): δ (ppm) = 9.15 (br, NH), 7.92 (d, *J* = 8.3 Hz, 2H, H-3/4), 7.74 (dd, *J* = 7.9, 1.4 Hz, 1H, H-2), 7.38 (d, *J* = 8.2 Hz, 2H, H-5/6), 7.27 - 7.11 (m, 3H, H-3'/4'/5'), 6.88 (br, NH), 4.17 (q, *J* = 7.1 Hz, 2H, $CH_2$-OEt), 3.68 (s, 2H, H-8), 1.51 (s, 9H, H-Boc), 1.26 (td, *J* = 7.1 Hz, 3H, $CH_3$-OEt).**$^{13}$C NMR** (101 MHz, $CDCl_3$): δ (ppm) = 171.1 (C-9), 165.5 (C-1), 154.7 (C-Boc), 138.3 (C-7), 133.2 (C-2), 131.0 (C-1'), 130.2 (C-6'), 129.7 (C-5/6), 127.8 (C-3/4), 126.1 (C-3'/4'/2'), 124.8 (C-5'), 81.5 ($C_{IV}$-Boc), 61.2 ($CH_2$-OEt), 41.4 (C-8), 28.4 ($CH_3$-Boc), 14.3 ($CH_3$-OEt). **ESI-MS** *m*/*z*=399.0 $[M+H]^+$, 397.0 $[M-H]^-$.

**2-(4-((2-((*tert*-Butoxycarbonyl)amino)phenyl)carbamoyl)phenyl)acetic acid (24).**

Compound **23** (455 mg, 1.14 mmol, 1 eq.) was solubilized in THF/$NaOH_{(aq)}$ 1M: 2/1 (5.5 ml), with NaOH (68.51 mg, 1.71 mmol, 1.5 eq.). The reaction mixture was stirred overnight at room temperature. After completion of the reaction, 50 ml of $H_2O$ were added and extracted with $CH_2Cl_2$ three times. The aqueous layer was acidified with $HCl_{aq}$ 1M until precipitation of the medium (pH=3-4) and extracted with EtOAc three times. The organic layers were dried over $Na_2SO_4$, filtered and concentrated under vacuum to afford compound **24** as a amorphous solid (376 mg, 1.01 mmol, y. 89%). **R*f*=** 0.31 ($CH_2Cl_2$/MeOH 9/1).

**$^1$H NMR** (400 MHz, $CDCl_3$): δ (ppm) = 9.19 (br, NH), 7.88 (d, *J* = 8.3 Hz, 2H, H-3/4), 7.70 (d, *J* = 7.2 Hz, 1H, H-2'), 7.32 (d, *J* = 8.4 Hz, 3H, H-5/6), 7.25 (dd, *J* = 7.1, 1.2 Hz, 2H, H-5'), 7.21 - 7.08 (m, 2H, H-3'/4'), 7.05 (s, NH), 3.67 (s, 2H, H-8), 1.48 (s, 9H, H-Boc). **$^{13}$C NMR** (101 MHz, $CDCl_3$): δ (ppm) = 175.6 (C-9), 166.0 (C-1), 154.9 (C-Boc), 137.8 (C-7), 133.1 (C-2), 130.6 (C-1'), 130.4 (C-6'), 129.7 (C-5/6), 127.8 (C-3/4), 126.0 (C-3'/4'), 124.8 (C-5'), 81.5 ($C_{IV}$-Boc), 41.0 (C-8), 28.4 ($CH_3$-Boc). **ESI-MS** *m*/*z*=371.0 $[M+H]^+$, 741.0 $[2M+H]^+$, 739.0 $[2M-H]^-$.

**2,5-Dioxopyrrolidin-1-yl 2-(4-((2-((*tert*-butoxycarbonyl)amino)phenyl)carbamoyl) phenyl) acetate (25).**

Compound **24** (199.3 mg, 0.54 mmol, 1 eq.), DCC (122.6 mg, 0.59 mmol, 1.1 eq.) et NHS (74.6 mg, 0.65 mmol, 1.2 eq.) were solubilized in $CH_3CN$ (11 ml). The reaction mixture was stirred at room temperature until completion of the reaction (6 h). The medium was filtered and concentrated *in vacuum* to afford **25** as an amorphous brown solid (251.6 mg, 0.54 mmol, y. quant.). **R*f*=** 0.36 ($CH_2Cl_2$/MeOH 97/3).

**$^1$H NMR** (400 MHz, $CDCl_3$): δ (ppm) = 9.22 (s, NH), 7.96 - 7.91 (d, *J* = 8.5 Hz, 2H, H-3/4), 7.72 (dd, *J* = 7.8, 1.5 Hz, 1H, H-2'), 7.43 (d, *J* = 8.5 Hz, 2H, H-5/6), 7.29 - 7.23 (dd, *J* = 7.8, 1.5 Hz, 1H, H-5'), 7.22 - 7.11 (m, 2H, H-3'/4'), 6.94 (br, NH), 3.99 (s, H-8), 2.82 (s, 4H, H-Suc), 1.50 (s, 9H, H-Boc). **$^{13}$C NMR** (101 MHz, $CDCl_3$): δ (ppm) = 169.0 (C-Suc), 166.4 (C-9), 165.3 (C-1), 154.7 (C-Boc), 135.4 (C-7), 133.9 (C-2), 130.8 (C-1'), 130.3 (C-2'), 129.7 (C-5/6), 128.1 (C-3/4), 126.2 (C-3'/4'), 126.0 (C-2'), 124.6 (C-5'), 81.5 ($C_{IV}$-Boc), 37.6 (C-8), 28.4 ($CH_3$-Boc), 25.70 (C-Suc). **ESI-MS** *m*/*z*= 468.2 $[M+H]^+$, 466.2 $[M-H]^-$.

**5-(2-(4-((2-((tert-Butoxycarbonyl)amino)phenyl)carbamoyl)phenyl)acetamido)pentanoic acid (26).**

Compound **25** (128.9 mg, 0.28 mmol, 1 eq.) was solubilized in 15 ml DMF and aminovaleric acid (49.7 mg, 0.42 mmol, 1.5 eq.) was added by portion wise. The reaction mixture was stirred at room temperature overnight and followed by HPLC. The mixture was concentrated *in vacuum* and purified by preparative HPLC using a linear gradient ($H_2O/CH_3CN$ + 0.1% TFA; 80/20 to 0/100) to afford **26** as a white powder (33.8 mg, 0.07 mmol, y. 25 %).

**$^1$H NMR** (400 MHz, MeOD): δ (ppm) = 7.92 (d, J = 8.3 Hz, 2H, H-3/4), 7.60 (dd, J = 7.4, 2.1 Hz, 1H, H-2'), 7.53 - 7.32 (m, 3H- H-5/6/5'), 7.28 - 7.16 (m, 2H, H-3'/4'), 3.59 (s, 2H, H-8), 3.21 (t, J = 6.6 Hz, 2H, C**$H_2$**NH linker), 2.30 (t, J = 7.1 Hz, 2H, C**$H_2$**COOH linker), 1.68 - 1.51 (m, 4H, H linker), 1.49 (s, 9H, NHBoc). **ESI-MS** *m*/*z*= 470.2 $[M+H]^+$, 929.4 $[2M+H]^+$.

**11-(2-(4-((2-((tert-Butoxycarbonyl)amino)phenyl)carbamoyl)phenyl)acetamido) undecanoic acid. (27)**

Compound **25** (148.8 mg, 0.32 mmol, 1 eq.) and aminoundecanoic acid (65.7 mg, 0.32 mmol, 1. eq.) were solubilized in 15 mL DMF and stirred at room temperature overnight. After completion of the reaction followed by HPLC, the mixture was concentrated *in vacuum* and purified by preparative HPLC using a linear gradient ($H_2O/CH_3CN$ + 0.1% TFA; 80/20 to 0/100) to afford **27** as a white powder (96.4 mg, 0.17 mmol, y. 53 %).

**$^1$H NMR** 1H NMR (400 MHz, $CDCl_3$): δ (ppm) = 9.32 (br, NH), 7.86 (dd, J = 8.0, 2.2 Hz, 2H, H-3/4), 7.64 (dd, J = 6.6, 3.5 Hz, 1H, H-2'), 7.34 - 7.28 (m, 3H, H-5/6/5'), 7.21 - 7.09 (m, 2H, H-3'/4'), 3.54 (s, 2H, H-8), 3.18 - 3.09 (t, J = 6.7 Hz, 2H, C**$H_2$**NH linker), 2.23 (t, J = 7.5 Hz, 2H, C**$H_2$**COOH linker), 1.55 (p, J = 7.3 Hz, 2H, C**$H_2$**$CH_2$NH linker), 1.46 (d, J = 1.6 Hz, 9H, NHBoc), 1.38 (m, 2H, C**$H_2$**$CH_2$COOH linker), 1.22 (m, 12H, H linker). **ESI-MS** *m*/*z*=554.3 $[M+H]^+$, 1107.6 $[2M+H]^+$.

**tert-Butyl (2-(4-(2-oxo-2-(prop-2-yn-1-ylamino)ethyl)benzamido)phenyl)carbamate. (28)**

Compound **24** (300.0 mg, 0.81 mmol, 1 eq.) was solubilized in 2.5 ml of $CH_2Cl_2$. DCC (253.7 mg, 1.21 mmol, 1.5 eq.), DMAP (9.89 mg, 0.08 mmol, 0.1 eq.) and propargylamine (46.8 mg, 55 μL, 0.85 mmol, 1.05 eq.) were added successively. The mixture was stirred at room temperature during 5 h, until completion of the reaction followed by TLC, then filtered and concentrated *in vacuum.* The crude was purified by manual flash chromatography ($CH_2Cl_2$/MeOH; from 100 % $CH_2Cl_2$ to 2% MeOH) to afford **28** as an amorphous solid (191.3 mg, 0.47 mmol, y. 56 %). **R*f***= 0.52 ($CH_2Cl_2$/MeOH 92/8).

**$^1$H NMR** (400 MHz, $CDCl_3$): δ (ppm) = 7.96 (d, J = 8.1 Hz, 2H, H-5/6), 7.83 (d, J = 7.9 Hz, 1H, H-2'), 7.38 (d, J = 8.1 Hz, 2H, H-3/4), 7.30 - 7.14 (m, 3H, H-3'/4'/5'), 4.03 (dd, J = 5.3, 2.6 Hz, 2H, $CH_2$ propargylamine), 3.65 (s, 2H, H-8), 2.21 (t, J = 2.6 Hz, 1H, H alkyn), 1.52 (s, 9H, NHBoc). **$^{13}$C NMR** (151 MHz, $CDCl_3$): δ (ppm) = 167.0 (C-1), 165.4 (C-9), 154.6 (C-Boc), 138.6 (C-2), 133.1 (C-7), 130.5 (C-1'), 130.3 (C-5/6), 128.0 (C-3/4), 126.0 (C-2'), 124.5 (C-6'), 81.2 ($C_{IV}$-Boc, 79.2 (C-alkyne), 71.7 (CH-alkyne), 43.0 (C-8), 29.3 ($CH_2$-propargylaminde), 28.3 ($CH_3$-Boc). **ESI-MS** *m*/*z*=408.2 $[M+H]^+$, 815.4 $[2M+H]^+$.

<u>Example 5: Synthesis of benzamide-based bivalent compounds</u>

* Compounds MuH2320

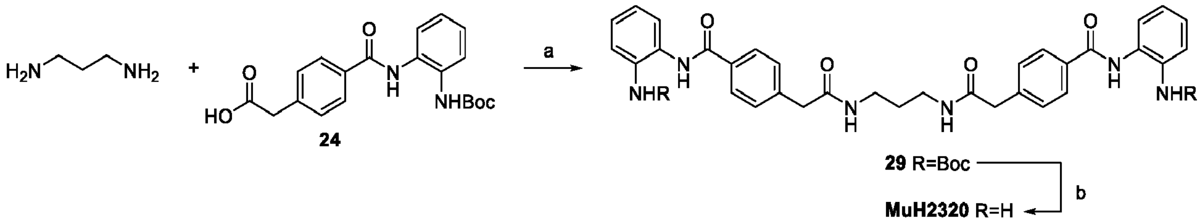


**24**

**29** R=Boc

**MuH2320** R=H

**(a)** PyBOP, TEA, DMF, RT, 20 h; **(b)** TFA, $CH_2Cl_2$, Tis, RT, 30 min.

* Compounds MuH2346

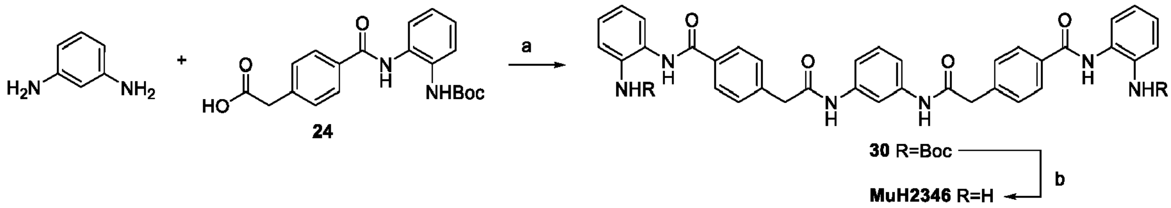


**(a)** HATU, DIPEA, DMF, RT, 16 h; **(b)** TFA, $CH_2Cl_2$, Tis, RT, 30 min.

* Compounds MuH2345

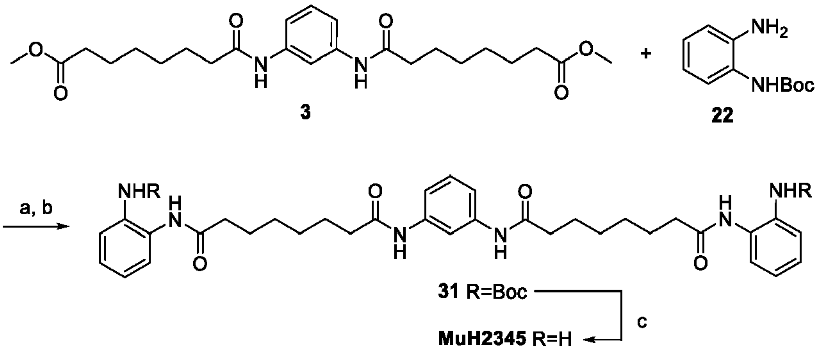


**(a)** KOH, $H_2O$/MeOH, RT, 5 h; **(b)** HATU, DIPEA, DMF, RT, 16 h; **(c)** TFA, $CH_2Cl_2$, Tis, RT, 30 min.

**di-*tert*-butyl(((4,4'-((propane-1,3-diylbis(azanediyl))bis(2-oxoethane-2,1-diyl))bis (benzoyl))bis(azanediyl)) bis(2,1-phenylene))dicarbamate. (29)**

Compound **24** (102.0 mg, 0.27 mmol, 4 eq.) and PyBOP (103.6 mg, 0.27 mmol, 4 eq.) were solubilized in DMF (130 μl) and stirred 5 min at room temperature. Then, diamine propane (5 mg, 5.7 μl, 0.07 mmol, 1 eq.) and TEA (96 μl) were added. The mixture was stirred at room temperature for 1 h, then at 40 °C for 4 h. After that, 0.2 eq of diamine were added (1.1 μl) and after 2 h temperature was increased to 60 °C and the medium was stirred overnight. The reaction was followed by HPLC and TLC and was stopped before completion of the reaction. The mixture was quenched with $H_2O$ and extracted with EtOAc three times. The organic layers were washed with $H_2O$ twice and brine, dried over $Na_2SO_4$, filtered, and concentrated under *vacuum.* The compound was purified by flash chromatography using a linear gradient ($CH_2Cl_2$/MeOH: 100/0 to 80/20) to separate mono-addition product from di-addition product. **29** was obtained as a yellow oil (11.8 mg, 0.015 mmol, y. 18 %). **R*f*=** 0.67 ($CH_2Cl_2$/MeOH 9/1). **$^1$H NMR** (400 MHz, $CDCl_3$): δ (ppm) = 9.23 (br, 2H, NH), 7.89 (d, J = 7.9 Hz, 4H, H-3/4), 7.71 (d, J = 7.3 Hz, 2H, H-2'), 7.34 (d, J = 8.1 Hz, 4H, H-5/6), 7.26 (m, 2H, H-5'), 7.16 (ddd, J = 6.4, 3.4, 2.2 Hz, 4H, H-3'/4'), 7.01 (br, 2H, NH), 6.23 (br, 2H, NH), 3.59 (s, 4H, H-8), 3.14 (m, 4H, H-A/C), 1.49 (s, 18H, CHBoc), H-B is obscured by water. **ESI-MS** *m*/*z*=779.4 $[M+H]^+$.

**4,4'-((Propane-1,3-diylbis(azanediyl))bis(2-oxoethane-2,1 -diyl))bis(N-(2-aminophenyl) benzamide). (MuH2320)**

Then, **29** was solubilized in 0.5 ml of TFA and stirred at room temperature 5 min. The crude was precipitated on ether and centrifugated to obtained **MuH2320** as a white powder (8.7 mg, 0.015 mmol, y. quant.). **$^1$H NMR** (400 MHz, MeOD): δ (ppm) = 8.04 - 7.96 (m, 4H, H-5/6), 7.53

**[0131]** - 7.39 (m, 12H-H-3/4/2'/3'/4'/5'), 3.61 (s, 4H, H-8), 3.23 (t, J = 6.8 Hz, 4H, H-A/C), 1.71 (p, J = 6.8 Hz, 2H, H-B). **$^{13}$C NMR** (101 MHz, MeOD): δ (ppm) = 173.3 (C-9), 169.0 (C-1), 142.1 (C-7), 132.9 (C-1'), 132.7 (C-6'), 130.5 (C-3/4), 130.0 (C-2), 129.4 (C-5/6), 128.8, 128.3, 127.6, 124.9 (C-2'/3'/4'/5'), 43.7 (C-8), 37.9 (C-A/C), 30.1 (C-B). **ESI-MS** *m*/*z*=579.3 $[M+H]^+$. **HRMS** Calcd for $C_{33}H_{35}N_6O_4$ 579.2714, Found 579.2707.

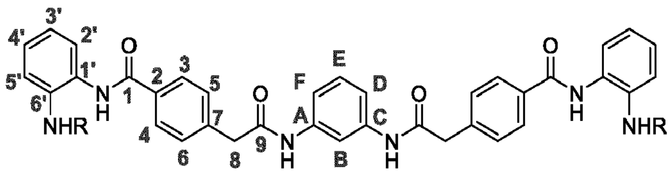

**Di-*tert*-butyl (((4,4'-((1,3-phenylenebis(azanediyl))bis(2-oxoethane-2,1-diyl))bis(benzoyl)) bis(azanediyl)) bis(2,1-phenylene))dicarbamate. (30)**

Compound **24** (108.4 mg, 0.29 mmol, 2.2 eq.) and HATU (116.0 mg, 0.31 mmol, 2.4 eq.) were solubilized in 1.4 ml of DMF. Then *p*-phenylenediamine (13.5 mg, 0.13 mmol, 1 eq.) was added to the mixture, followed by DIPEA (72 µl, 0.42 mmol, 3.2 eq.) and stirred at room temperature overnight. After completion of the reaction followed by TLC, the mixture was diluted with $H_2O$ and extracted three times with $CH_2Cl_2$. The organic phases were washed with saturated NaHCOs solution once, brine twice, dried over $Na_2SO_4$, filtered, and concentrated under *vacuum.* The crude mixture was filtered through a pad of silica eluted with $CH_2Cl_2$/MeOH: 95/5 to afford **30** as a brown oil (83.0 mg, 0.10 mmol, y. 80%). **R*f*=** 0.25 ($CH_2Cl_2$/MeOH 95/05). **$^1$H NMR (400 MHz, $CDCl_3$)** δ 9.30 (br, 2H, NH), 9.05 (br, 2H, NH), 8.00 (s, 2H), 7.50 (m, 10H), 7.08 (m, 10H), 3.55 (s, 4H, H-8), 1.46 (s, 18H, CHBoc). **ESI-MS** *m*/*z*=813.4 $[M+H]^+$.

**4,4'-((1,3-Phenylenebis(azanediyl))bis(2-oxoethane-2,1-diyl))bis(N-(2-aminophenyl) benzamide). (MuH2346)**

Compound **17** (42.5 mg, 0.05 mmol, 1 eq.) was solubilized in 1 ml TFA/$CH_2Cl_2$/Tis: (50/45/5) and stirred at room temperature 30 min. After completion of the reaction, TFA was neutralized with 3 ml pyridine/MeOH 20%, precipitated in diethyl ether and centrifugated (4500 RPM, 15 min). The pellet was dried to obtain **MuH2346** as a brown solid (34.9 mg, 0.05 mmol, y. quant.). **$^1$H NMR** (600 MHz, DMSO): δ (ppm) = 10.22 (br, 2H, NH-9), 9.77 (br, 2H, NH-1), 8.01 - 7.86 (m, 5H, H-3/4/F), 7.47 (d, J = 8.2 Hz, 4H, H-5/6), 7.27 (dd, J = 7.4, 1.9 Hz, 2H, H-B/D), 7.22 (m, 3H, H-C/2'), 7.05 (td, J = 7.6, 1.6 Hz, 2H, H-4'), 6.90 (dd, J = 8.0, 1.4 Hz, 2H, H-5'), 6.76 (t, J = 7.6 Hz, 2H, H-3'), 3.73 (s, 4H, H-8). **$^{13}$C NMR** (151 MHz, DMSO): δ (ppm) = 169.1 (C-9), 165.8 (C-1), 140.4 (C-1'), 140.2 (C-7), 139.9 (C-A/E), 133.2 (C-2), 129.5 (C-5/6), 129.4 (C-F), 128.3 (C-3/4), 127.1 (C-2'), 127.0 (C-4'), 125.7 (C-6'), 119.0 (C-3'), 118.1 (C-5'), 114.8 (C-B/D), 110.8 (C-C), 43.6 (C-8). **HRMS** Calcd for $C_{36}H_{33}N_6O_4$ 613.2558, Found 613.2555.

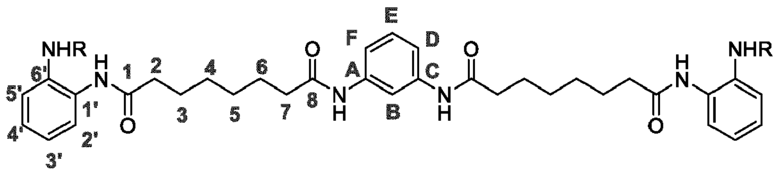

**Di-tert-butyl (((8,8'-(1,3-phenylenebis(azanediyl))bis(8-oxooctanoyl))bis(azanediyl))bis (2,1-phenylene))dicarbamate. (31).**

Compound **3** (242.0 mg, 0.54 mmol) was diluted in 2 ml of MeOH and 2 ml of KOH 3M in $H_2O$, and stirred at room temperature. After 5 h, the reaction mixture was poured in iced water and filtered to obtain the saponified compound. **$^1$H NMR** (400 MHz, MeOD): δ (ppm) = 7.84 (t, J = 2.0 Hz, 1H, H-B), 7.46 - 7.13 (m, 3H, H-D/E/F), 2.36 (t, J = 7.5 Hz, 4H, H-7), 2.29 (t, J = 7.4 Hz, 4H, H-2), 1.85 - 1.50 (m, 8H, H-3/6), 1.39 (m, J = 3.7 Hz, 8H, H-4/5). **$^{13}$C NMR** (101 MHz, $CDCl_3$): δ = 178.6 (C-8), 177.2 (C-1), 142.9 (C-A/C), 132.5 (C-E), 119.5 (C-F/D), 115.8 (C-B), 40.4 (C-7), 37.3 (C-2), 32.4 (C-4/5), 29.3 (C-6), 28.4 (C-3). **ESI-MS** *m*/*z*=421.2 $[M+H]^+$. Then, the bis-carboxylic acid intermediate (60.0 mg, 0.14 mmol, 1 eq.) and HATU (117.7 mg, 0.31 mmol, 2.2 eq.) were solubilized in 1.4 ml of DMF. Then, compound **22** (59.4 mg, 0.29 mmol, 2 eq.) was added to the mixture, followed by DIPEA (74 µl, 0.42 mmol, 3 eq.), and the reaction mixture was stirred at room temperature overnight. After completion of the reaction followed by HPLC, the mixture was diluted with $H_2O$ and extracted three times with $CH_2Cl_2$. The organic phase was washed with saturated NaHCOs solution once, brine once, dried over $Na_2SO_4$, filtered, and concentrated under *vacuum.* The compound was purified by flash chromatography using a linear gradient ($CH_2Cl_2$/MeOH: 100/0 to 90/10) to afford **31** as an oil (20.9 mg, 0.03 mmol, y. 18%). **R*f*=** 0.7 ($CH_2Cl_2$/MeOH 95/05). **$^1$H NMR** (400 MHz, $CDCl_3$): δ (ppm) = 8.61 (br, 2H, NH-9), 8.36 (br, 2H, NH-1), 7.72 (s, 1H, H-B), 7.43 (dd, J = 8.0, 1.5 Hz, 2H, H-F/D), 7.35 (m, 3H, H-E/5'), 7.19 (m, 2H, H-3'), 7.11 (m, 2H, H-2'), 7.04 (td, J = 7.6, 1.6 Hz, 2H, H-4'), 2.42 - 2.13 (m, 8H, H-7/2), 1.60 (q, J = 7.4 Hz, 8H, H-3/6), 1.48 (s, 18H, $CH_3$-Boc), 1.26 (m, 8H, H-4/5). **ESI-MS** *m*/*z*=801.3 $[M+H]^+$.

***N*[1],*N*[1']-(1,3-Phenylene)bis(N8-(2-aminophenyl)octanediamide). (MuH2345)**

Compound **31** (20.9 mg, 0.03 mmol, 1 eq.) was solubilized in 1 ml of TFA/$CH_2Cl_2$/Tis: (50/45/5) and stirred at room temperature 30 min. The crude mixture was neutralized with 3 ml of pyridine/MeOH (2/8) and concentrated *in vacuum.* The residue was precipitated in diethyl ether and centrifugated (15 min, 4500 rpm) to afford **MuH2345** as a pink powder (12.5 mg, 0.02 mmol, y. 80 %). **$^1$H NMR** (600 MHz, DMSO): δ (ppm) = 9.84 (br, 2H, NH-9), 9.19 (br, 2H, NH-1), 7.92 (s, 1H, H-B), 7.26 (d, J = 8.1, 2H, H-F/D), 7.16 (m, 3H, H-E/5'), 6.93 (m, 2H, H-3'), 6.77 (m, 2H, H-2'), 6.61 (m, 2H, H-4'), 2.30 (m, 8H, H-2/7), 1.59 (m, 8H, H-3/6), 1.34 (m, 8H, H-4/5). **$^{13}$C NMR** (151 MHz, DMSO): δ (ppm) = 171.7 (C-1/8), 140.0 (C-A/1'), 129.2 (C-5'), 126.2 (C-3'), 125.8 (C-E), 125.0 (C-6'), 117.9 (C-4'), 117.2 (C-2'), 114.3 (C-F/D), 110.5 (C-B), 36.8 and 36.2 (C-2/3), 29.0 (C-4/5), 25.6 (C-3/6). **HRMS** Calcd for $C_{34}H_{45}N_6O_4$ 601.3497, Found 601.3488.

<u>Example 6: Synthesis of benzamide-based multivalent compounds, with cyclic peptides</u>

* Compounds MuH2122 and MuH2124.

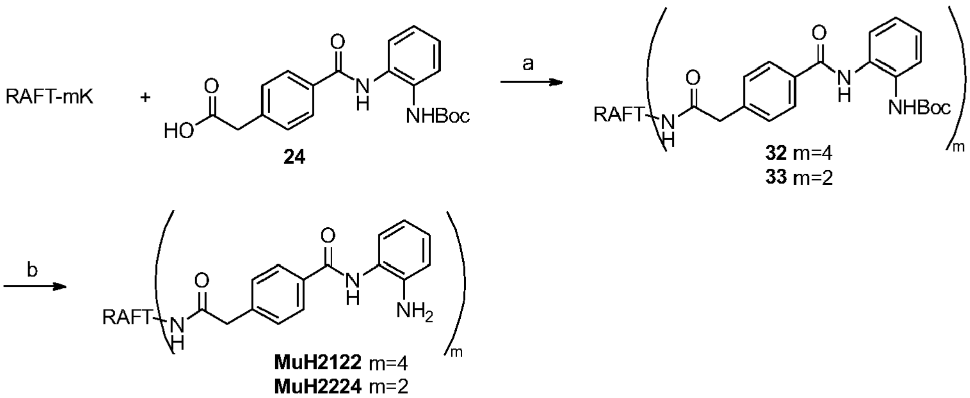


**24**

**32** m=4
**33** m=2

**MuH2122** m=4
**MuH2224** m=2

**(a)** HATU, DIPEA, DMF, RT; **(b)** TFA, $CH_2Cl_2$, Tis, RT

* Compounds MuH2220 and MuH2221

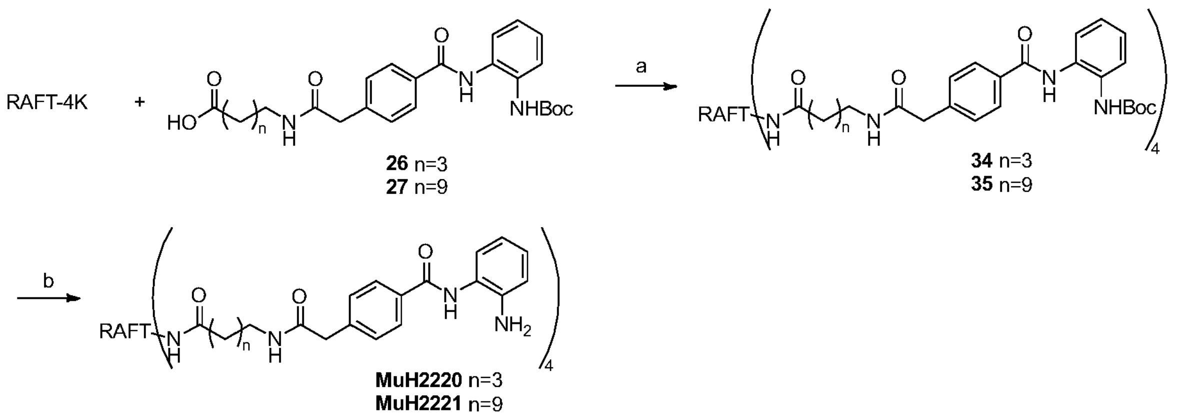


**26** n=3
**27** n=9

**34** n=3
**35** n=9

**MuH2220** n=3
**MuH2221** n=9

**(a)** HATU, DIPEA, DMF, RT; **(b)** TFA, $CH_2Cl_2$, Tis, RT
RAFT: SEQ ID NO: 5; RAFT-4K: SEQ ID NO: 10 and RAFT-4K($N_3$): SEQ ID NO: 41

* Compound MuH2222 -

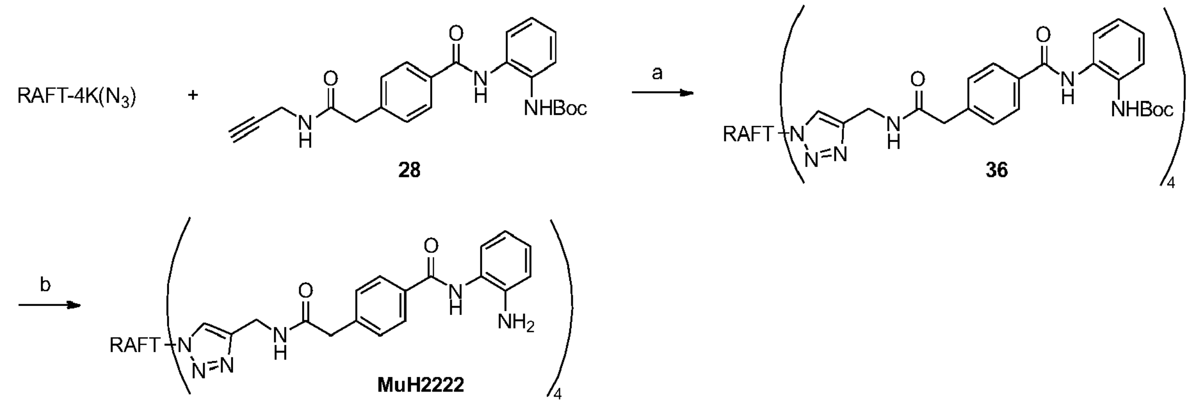


28

36

MuH2222

**(a)** $CuSO_4.5H_2O$, NaAsc, tBuOH/$H_2O$, RT; **(b)** TFA, $H_2O$, RT

**RAFT-(tert-butyl (2-(4-(2-X-2-oxoethyl)benzamido)phenyl)carbamate)$_4$. (32)**

RAFT-4K (10 mg, 10.3 μmol, 1 eq.), compound **24** (38.2 mg, 0.1 mmol, 10 eq) and HATU (39.1 mg, 0.1 mmol, 10 eq.) were solubilized in DMF (640 μl). Then, DIPEA (45 μl) was added, and the reaction mixture was stirred at room temperature overnight. After completion of the reaction, the solvent was evaporated *in vacuum*. The crude product was used in the next step without further purification. **ESI-MS** m/z=1187.2 $[M+2H]^{2+}$.

**RAFT-(4-(2-X-2-oxoethyl)-N-(2-aminophenyl)benzamide)$_4$. (MuH2122)**

**32** was solubilized in 1 ml TFA/$CH_2Cl_2$ (25/75). After completion of the deprotection, the reaction mixture was concentrated and directly purified by semi-preparative HPLC using a linear gradient ($H_2O/CH_3CN$; 95/5 to 0/100) to afford **MuH2122** as a white powder (8.2 mg, 4.2 μmol, y. 40%). **ESI-MS** *m/z*=1973.2 $[M+H]^+$, 987.4 $[M+2H]^{2+}$, 1971.3 $[M-H]^-$, 985.1 $[M-2H]^{2-}$. **HRMS** Calcd for $C_{104}H_{128}N_{22}O_{18}$ 986.4883, Found 986.4882.

**RAFT-(tert-butyl (2-(4-(2-X-2-oxoethyl)benzamido)phenyl)carbamate)$_2$. (33)**

RAFT-2K (10.3 mg, 10.3 μmol, 1 eq.), compound **24** (21.9 mg, 0.06 mmol, 5 eq.), and HATU (22.6 mg, 0.06 mmol, 5 eq.) were solubilized in DMF (420 μl). Then, DIPEA (20 μl) was added, and the reaction mixture was stirred at room temperature during 2.5 h. After completion of the reaction, the solvent was evaporated *in vacuum*. The residue was purified by preparative HPLC using a linear gradient ($H_2O/CH_3CN$ + 0.1 % TFA; 90/10 to 70/30) to obtain the protected divalent inhibitor as a white powder (12.9 mg, 7.7 μmol, y. 75 %). **ESI-MS** *m/z*=1553.5 $[M+H]^+$.

**RAFT-(4-(2-X-2-oxoethyl)-N-(2-aminophenyl)benzamide)$_2$. (MuH2224)**

**33** (12.9 mg, 7.7 μmol, 1 eq.) was solubilized in 3 ml TFA/$CH_2Cl_2$/Tis (50/25/25) and stirred at room temperature. After 1 h the deprotected peptide was precipitated on ether and centrifugated twice to obtain **MuH2224** as a white powder (6.8 mg, 5.0 μmol, y. 42 %). **ESI-MS** *m/z*=1353.5 $[M+H]^+$, 677.8 $[M+2H]^{2+}$. **HRMS** Calcd for $C_{68}H_{89}N_{16}O_{14}$ 1353.6739, Found 1353.6729.

**RAFT-(tert-butyl(2-(4-(2-((5-X-5-oxopentyl)amino)-2-oxoethyl)benzamido)phenyl) carbamate)$_4$. (34)**

RAFT-4K (6.5 mg, 6.7 μmol, 1 eq.), compound **26** (25.3 mg, 0.05 mmol, 8 eq), HATU (25.7 mg, 0.07 mmol, 10 eq.) were solubilized in DMF (240 μl). Then, DIPEA (23 μl) was added, and the reaction mixture was stirred at room temperature overnight. After completion of the reaction, the solvent was evaporated *in vacuum*. The residue was purified by preparative HPLC using a linear gradient ($H_2O/CH_3CN$ + 0.1 % TFA; 80/20 to 0/100) to obtain the protected tetravalent inhibitor as a white powder (11.9 mg, 4.3 μmol, y. 64 %). **ESI-MS** *m/z*=1385.4 $[M+2H]^{2+}$.

**RAFT-(4-(2-((5-X-5-oxopentyl)amino)-2-oxoethyl)-N-(2-aminophenyl)benzamide)$_4$. (MuH2220)**

**34** (11.9 mg, 4.3 μmol, 1 eq.)was solubilized in 4 ml TFA/$CH_2Cl_2$/Tis (50/40/10) and stirred at room temperature. After 1 h the deprotected peptide was precipitated on ether and centrifugated. The pellet was purified preparative HPLC using a linear gradient ($H_2O/CH_3CN$ + 0.1 % TFA; 80/20 to 0/100) to afford **MuH2220** as a white powder (2.1 mg, 0.9 μmol,

y. 13 %). **ESI-MS** *m/z*=1185.2 $[M+2H]^{2+}$, 790.7 $[M+3H]^{3+}$. **HRMS** Calcd for $C_{124}H_{164}N_{26}O_{22}$ 1184.6551, Found 1184.6249.

**RAFT-(tert-butyl(2-(4-(2-((11-X-11-oxoundecyl)amino)-2-oxoethyl)benzamido)phenyl) carbamate)$_4$. (35)**

RAFT-4K (10.0 mg, 10.4 μmol, 1 eq.), compound **27** (57.7 mg, 0.1 mmol, 10 eq), HATU (39.4 mg, 0.1 mmol, 10 eq.) were solubilized in DMF (370 μl). Then, DIPEA (35 μl) was added, and the reaction mixture was stirred at room temperature 8 h. After completion of the reaction, the solvent was evaporated *in vacuum.* The crude product was used in the next step without further purification. **ESI-MS** *m/z*=1553.4 $[M+2H]^{2+}$, 1036.1 $[M+3H]^{3+}$.

**RAFT-(4-(2-((11-X-1-oxoundecyl)amino)-2-oxoethyl)-N-(2-aminophenyl)benzamide)$_4$. (MuH2221)**

The crude **35** was solubilized in 3 mL TFA/$CH_2Cl_2$/Tis (60/30/10) and stirred at room temperature 30 min. The mixture was concentrated *in vacuum* and purified by preparative HPLC using a linear gradient ($H_2O/CH_3CN$ + 0.1 % TFA; 80/20 to 0/100) to afford **MuH2224** as a white powder (4.2 mg, 1.6 μmol, y. 15 %). **ESI-MS** *m/z*=1353.3 $[M+2H]^{2+}$, 902.7 $[M+3H]^{3+}$. **HRMS** Calcd for $C_{148}H_{213}N_{26}O_{22}$ 902.2110, Found 902.2098.

**RAFT-(tert-butyl(2-(4-(2-(((1-X-1H-1,2,3-triazol-4-yl)methyl)amino)-2-oxoethyl) benzamido)phenyl)carbamate)$_4$. (36)**

Solutions of NaAsc 0.09 M in $H_2O$ and $CuSO_4.H_2O$ 0.11 M in $H_2O$ were previously prepared. **RAFT-4K($N_3$)** (10 mg, 9.4 μmol, 1 eq.) and compound **28** (23.5 mg, 0.06 mmol, 6 eq.) were solubilized in tBuOH (200 μl). $CuSO_4.H_2O_{(aq)}$ (65 μl) and $NaAsc_{(aq)}$ (65 μl) were added, and the mixture was stirred at room temperature. After completion of the reaction, followed by HPLC (1 h), the mixture was concentrated *in vacuum* and purified by semi-preparative HPLC using a linear gradient ($H_2O/CH_3CN$ + 0.1 % TFA; 80/20 to 0/100) to afford the protected peptide as a white powder (9.1 mg, 3.4 μmol, y. 36 %). **ESI-MS** *m/z*=1349.4 $[M+2H]^{2+}$, 899.9 $[M+3H]^{3+}$.

**RAFT-(*N*-(2-aminophenyl)-4-(2-(((1-X-1H-1,2,3-triazol-4-yl)methyl)amino)-2-oxoethyl) benzamide)$_4$. (MuH2222)**

**36** (9.1 mg, 3.4 μmol, 1 eq.). was solubilized in 2 ml of TFA/$H_2O$ (95/5) and stirred at room temperature. After 30 min, the mixture was freeze-dried to afford **MuH2222** as a white powder (6.8 mg, 3.0 μmol, y. 87 %). **ESI-MS** *m/z*=1149.3 $[M+2H]^{2+}$, 766.7 $[M+3H]^{3+}$. **HRMS** Calcd for $C_{116}H_{140}N_{34}O_{18}$ 1148.5537, Found 1148.5522.

Example 7: control compound

**RAFT-(4-pentyn-1-ol)$_4$.**

In a vial, RAFT-$N_3$ (5 mg, 4.7 μmol, 1 eq.), 4-pentyn-1-ol (2.4 mg, 28 μmol, 6 eq.), sodium ascorbate (0.9 mg, 4.7 μmol, 1 eq.) and pentahydrate copper sulfate (1.2 mg, 4.7 μmol, 1 eq.) were solubilized in 170 μl tBuOH/phosphate buffer 500 mM pH=7.4 (6/4). The reaction mixture was heated at 50°C and stirred overnight. Then, the medium was filtered and freeze-dried. The residue was purified by semi-preparative HPLC using a linear gradient ($H_2O/CH_3CN$; 95/5 to 30/70) to afford **11** as a white powder (4.1 mg, 2.9 μmol, y. 63%). **ESI-MS** *m/z*=1403.3 $[M+H]^+$, 702. 5 $[M+2H]^{2+}$.

**Example 8:** HDAC inhibition potency of hydroxamic acid-based multivalent HDACi

Compound HDAC inhibition activities were determined with a fluorescence-based assay in solution, using HeLa nuclear extracts purchased from IpraCell. HeLa nuclear extracts were incubated for 45 min at 37 °C in a solution of TosGPK(Ac)-AMC peptide substrate (43 μM final concentration) with different concentration of cascade diluted inhibitors (from 32 nM to 100 μM final concentration) or DMSO as control in enzyme buffer (25 mM TrisHCl pH 8, 140 mM NaCl, 3 mM KCl, 1 mM $MgCl_2$, final concentrations). Trypsin solution was then added (0.29 μg/μl final concentration) to release AMC adduct of non-acetylated peptides. After 15 min incubation at room temperature 150 μl of absolute ethanol were added and AMC-fluorescence level was measured on a Safas Xenius spectrofluorometer ($\lambda_{exc}$./$\lambda_{em}$. = 380/460 nm). Inhibition percentages for a concentration range were then used to calculate the half maximal effective concentration ($EC_{50}$) (average of at least 2 experiments) for the compound of interest by using the nonlinear regression with sigmoidal dose-response (variable slope) tool of GraphPad Prism software.

$EC_{50}$ Inhibition values of benzamide-based multivalent HDACi are combined the following table:

| Compound | Platform | Valence | Linker | Zn chelating group | $EC_{50}$ (μM) |
|---|---|---|---|---|---|
| **SAHA** | - | - | - | Hydroxamic acid | 0.85 ± 0.40 |
| **MLo2125** | - | - | - | Hydroxamic acid | 7.04 |
| **MuH2123** | RAFT | 4 | Triaz-C4 | Hydroxyl | Inact |
| **MuH2120** | RAFT | 4 | C6 | Hydroxamic acid | 2.84 |
| **MuH2121** | RAFT | 4 | C6 | Hydroxamic acid | 5.76 |
| **MuH2218** | RAFT | 4 | Triaz-C4 | Hydroxamic acid | n.d. |
| **MuH2321** | Triaz | 2 | C6 | Hydroxamic acid | **0.23 ± 0.16** |
| **MuH2322** | Arom-o | 2 | C6 | Hydroxamic acid | **0.52 ± 0.11** |
| **MuH2323** | Arom-*p* | 2 | C6 | Hydroxamic acid | **0.48 ± 0.32** |
| **MuH2324** | Arom-*m* | 2 | C6 | Hydroxamic acid | **0.06 ± 0.01** |
| **MuH2344** | Arom | 4 | C6 | Hydroxamic acid | 2.25 ± 0.65 |
| **MuH2357** | RAFT | 2 | -*o*-Ph-C6 | Hydroxamic acid | **0.23 ± 0.07** |

These data show that independently of the linker lengths or natures, and independently of the platforms, tetravalent HDACi are less potent than the monovalent inhibitor SAHA. Very interestingly, divalent hydroxamic acid featured by a triazol or an aromatic or a peptide platform are strongly more potent than the monovalent inhibitor SAHA. Compound MuH2324 show a 14-fold increase in activity compare to the SAHA.

<u>Example 9: HDAC inhibition potency of benzamide-based multivalent HDACi</u>

HDAC inhibition activity of benzamide-based multivalent HDACi were carried out using the procedure described in Example 8. ECso Inhibition values of benzamide-based multivalent HDACi are combined in the following table:

| Compound | Platform | Valence | Linker | Zn chelating group | $EC_{50}$ (μM) |
|---|---|---|---|---|---|
| **MS275** | - | - | - | Benzamide | 7.23 ± 2.07 |
| **MuH2122** | RAFT | 4 | C0 | Benzamide | **1.59 ± 0.87** |
| **MuH2220** | RAFT | 4 | C4 | Benzamide | **1.22 ± 0.70** |
| **MuH2222** | RAFT | 4 | TriazC1 | Benzamide | **1.76 ± 0.89** |
| **MuH2224** | RAFT | 2 | Phenyl | Benzamide | 7.81 ± 5.27 |
| **MuH2320** | Butyl | 2 | C3 | Benzamide | 9.21 ± 2.55 |
| **MuH2345** | Arom-*m* | 2 | C6 | Benzamide | 31.54 ± 29.92 |
| **MuH2346** | Arom-*m* | 2 | Phenyl | Benzamide | n.d. |

These data show that divalent benzamid-based HDACi are less potent than the corresponding MS275 monovalent inhibitor. Surprisingly and contrarily to the hydroxamic-acid based HDACi, an increased potency is observed for the tetravalent benzamide-based HDACi with a 4- to 6-fold increased activity.

## Claims

1. A compound of formula R-A-R, wherein R is a C4-C6 alcohol or an hydroxamic acid of formula (I)

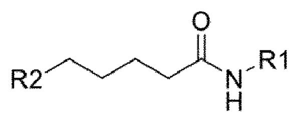

wherein

R1 is OH,
R2 is a C1-C12 alkyl, substituted by an amide group a triazol group, an amide group, or an acid group, a phenyl group, or a combination thereof,

and wherein A is a C5-C11 alkyl,a group $(CH_2\text{-}CH_2O\text{-})_n$ wherein n is a integer from 1 to 40, α,ω-diamino- or α,ω-diazido-aikyl, a triazol or a compound comprising two amines or azide groups, or any group allowing the covalent link with both R residues.

2. The compound according to claim 1, wherein A is chosen from the group consisting in a C5-C11 alkyl, a group $(CH2\text{-}CH2O\text{-})_n$ wherein n is a integer from 1 to 40, α,ω-diamino- or α,ω-diazido-aikyl, a triazol, an aryl diamine, a C1-C3 alkyl diamine or diacide, a peptide, cyclic or not, comprising two reactive amino acids chosen from lysine, serine, aspartic acid, glutamic acid, cysteine, threonine, and tyrosine..

3. The compound according to claim 2, wherein the peptide is a 6-12-amino acids peptide, in particular a cyclic peptide, preferably a 10-amino acids cyclic peptide comprising 2 lysines.

4. The compound according to claim 1 or 2, wherein R is chosen from:

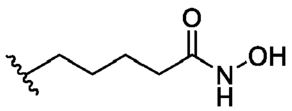 ; 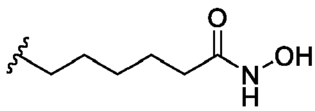 ; 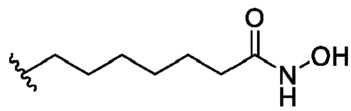 ;

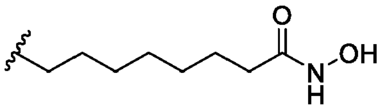 ; 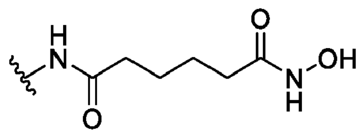

; 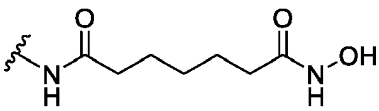 ; 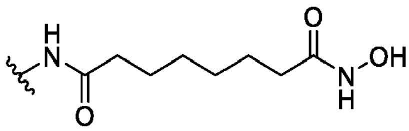 ;

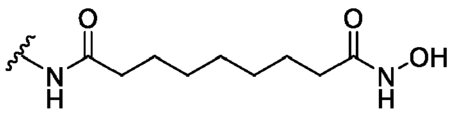

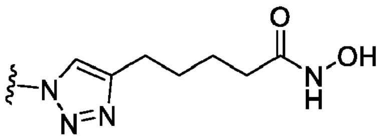

; 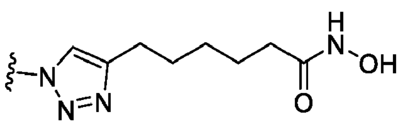 ; 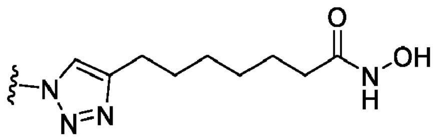 ;

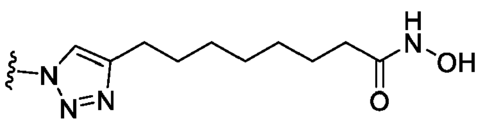

;

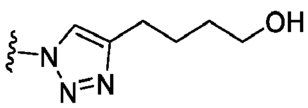 ; and 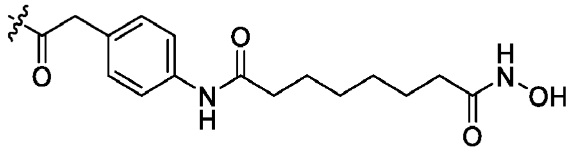

**5.** The compound according to anyone of claims 1 to 4, wherein A is chosen from:

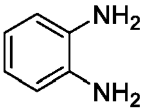 ; 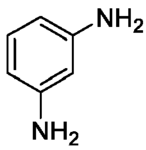 ; 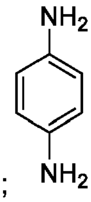 ; 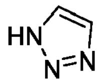

or a 10-amino acids peptide, preferably a cyclic peptide, having a sequence as set force in sequence XXXXK/RXXXXK/R.

**6.** The compound according to claim 5, wherein the 10-amino acids peptide has a sequence as set force in SEQ ID NO: 1 to SEQ ID NO: 8.

**7.** The compound according to anyone of claims 1 to 6, wherein the compound has one of the following formulas:

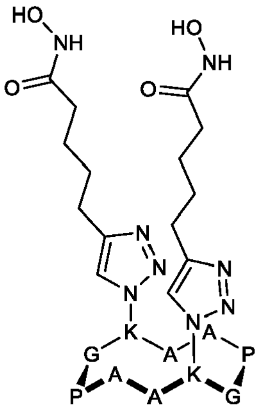 ; 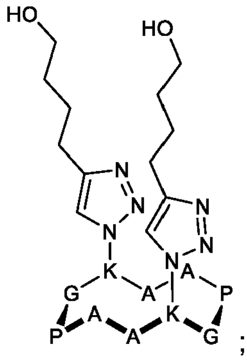 ; 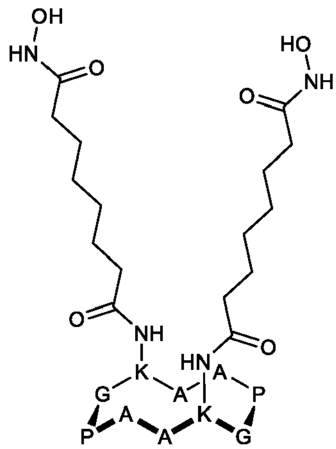 ;

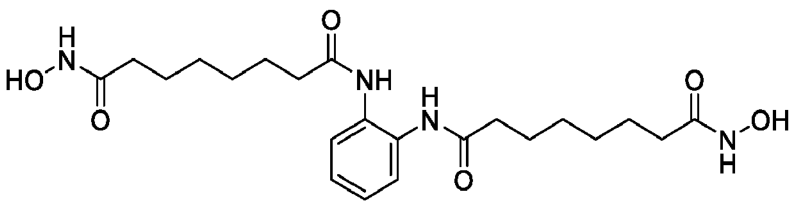

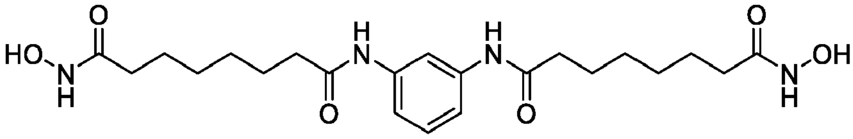

;

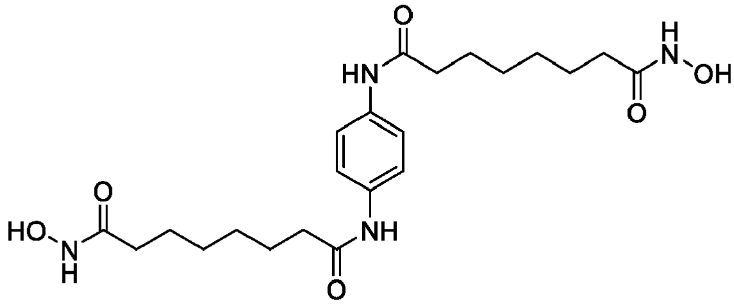

;

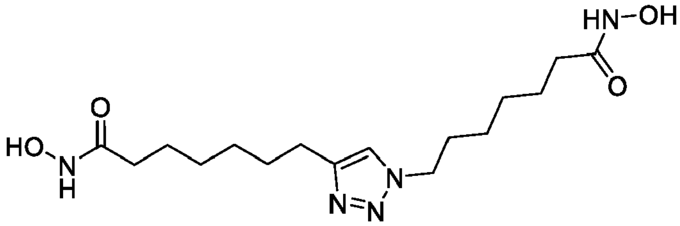

;

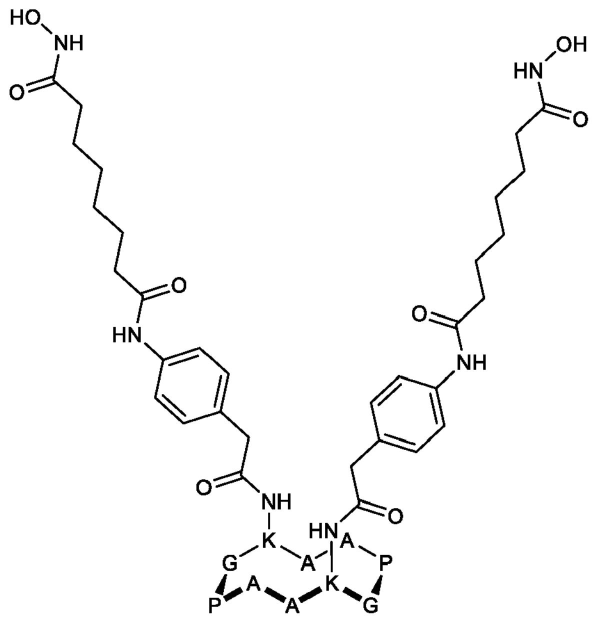

;

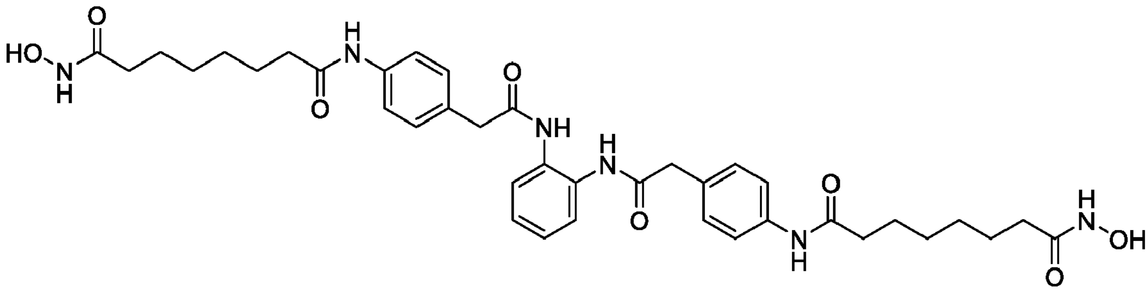

;

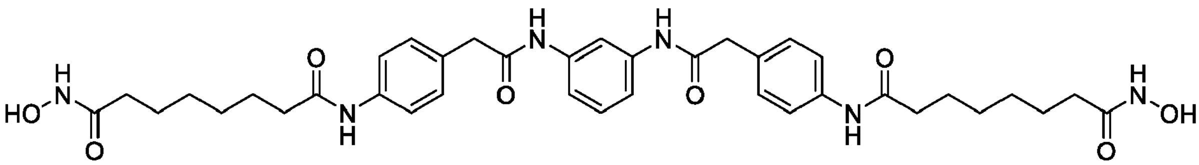

; and

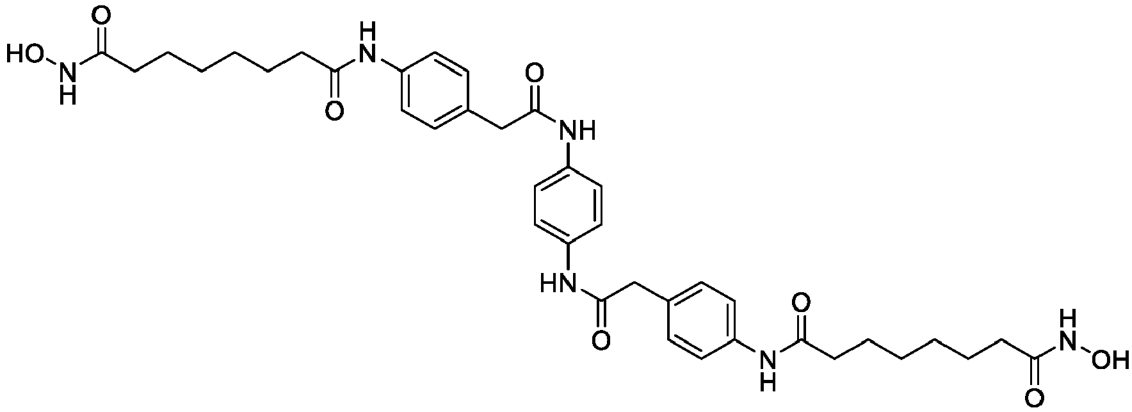

**8.** A pharmaceutical composition comprising as active substance the compound according to anyone of claims 1 to 7, in association with a pharmaceutically acceptable carrier.

**9.** The compound according to anyone of claims 1 to 7, for is use as medicament.

**10.** The compound according to anyone of claims 1 to 7, for treating a pathology involving a deregulation of a Histone deacetylase or HDAC.

**11.** The compound for its use according to claim 10, wherein the pathology involving a deregulation of a Histone deacetylase or HDAC is cancer, in particular hematological cancer or solid tumor, Crohn disease, schizophrenia, HIV infection, Parkinson's and Alzheimer's diseases and *Plasmodium falciparum* and *Schistosoma mansoni* infection.

[Fig. 1]

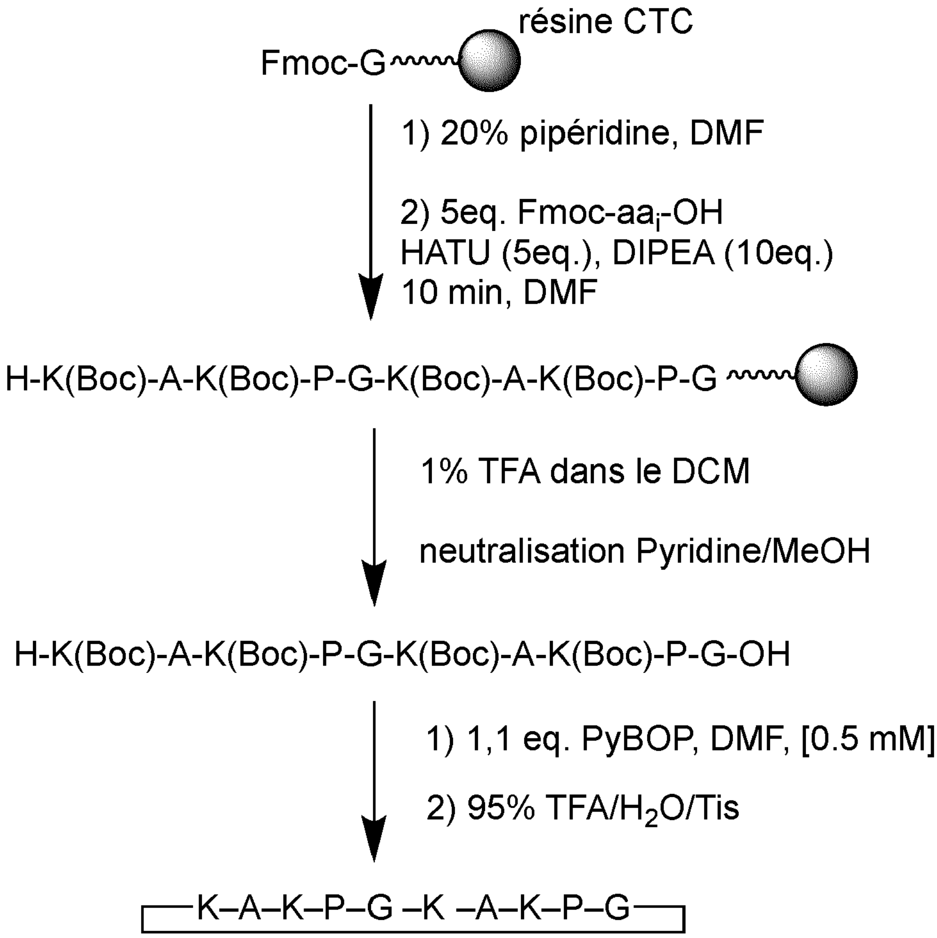


***SEQ ID NO: 5***

[Fig. 2]

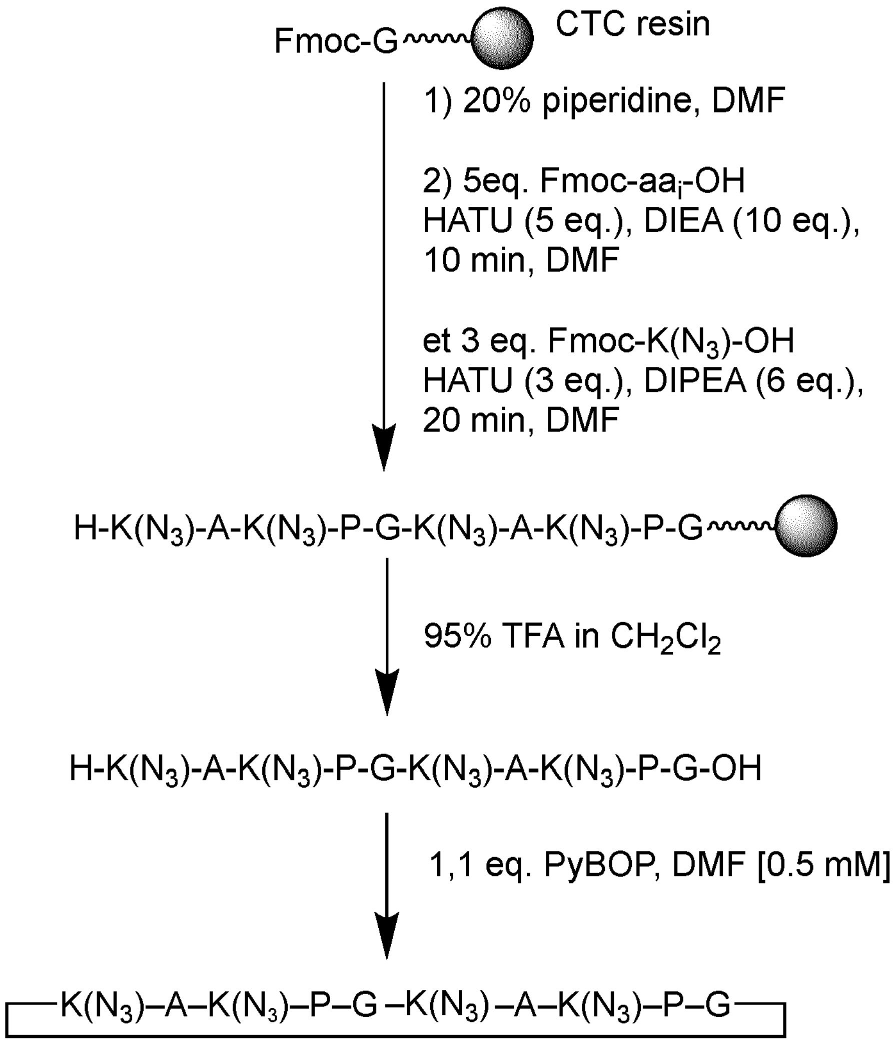


SEQ ID NO: 41

Europäisches Patentamt
European Patent Office
Office européen des brevets

# EUROPEAN SEARCH REPORT

Application Number
EP 23 30 7357

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2008/091349 A1 (HARVARD COLLEGE [US]; DANA FARBER CANCER INST INC [US] ET AL.) 31 July 2008 (2008-07-31)<br>* the whole document *<br>* claims 39-58; figures 1-10,23-24 *<br>----- | 1-11 | INV.<br>A61P31/00<br>A61P35/00<br>C07C259/06<br>C07C275/24<br>C07C323/44<br>C07D249/04<br>C07D413/12<br>C07K7/64 |
| X | SUN QIAO ET AL: "Design, synthesis, and biological evaluation of novel histone deacetylase 1 inhibitors through click chemistry",<br>BIOORGANIC & MEDICINAL CHEMISTRY LETTERS, ELSEVIER, AMSTERDAM NL,<br>vol. 23, no. 11, 4 April 2013 (2013-04-04), pages 3295-3299, XP028535157,<br>ISSN: 0960-894X, DOI: 10.1016/J.BMCL.2013.03.102<br>* the whole document *<br>* table 2; compound 28 *<br>----- | 1-11 | |
| X | US 2013/065963 A1 (KOZIKOWSKI ALAN P [US] ET AL) 14 March 2013 (2013-03-14)<br>* the whole document *<br>* page 14; claim 100; example 17 *<br>----- | 1-11 | TECHNICAL FIELDS SEARCHED (IPC)<br>C07K<br>A61P<br>C07C<br>C07D |
| A | DUMY P ET AL: "A Convenient Synthesis of Cyclic Peptides as Regioselectively Addressable Functionalized Templates (RAFT)",<br>TETRAHEDRON LETTERS, ELSEVIER, AMSTERDAM, NL,<br>vol. 36, no. 8, 20 February 1995 (1995-02-20), pages 1255-1258, XP004028744,<br>ISSN: 0040-4039, DOI: 10.1016/0040-4039(94)02481-P<br>* the whole document *<br>----- | 1-11 | |

The present search report has been drawn up for all claims

| Place of search | Date of completion of the search | Examiner |
|---|---|---|
| Munich | 14 May 2024 | Cervigni, S |

3

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
..........................................
& : member of the same patent family, corresponding document

EPO FORM 1503 03.82 (P04C01)

## ANNEX TO THE EUROPEAN SEARCH REPORT ON EUROPEAN PATENT APPLICATION NO. EP 23 30 7357

This annex lists the patent family members relating to the patent documents cited in the above-mentioned European search report. The members are as contained in the European Patent Office EDP file on
The European Patent Office is in no way liable for these particulars which are merely given for the purpose of information.

14-05-2024

| Patent document cited in search report | Publication date | Patent family member(s) | Publication date |
|---|---|---|---|
| WO 2008091349 A1 | 31-07-2008 | AU 2007345292 A1 | 31-07-2008 |
| | | CA 2642273 A1 | 31-07-2008 |
| | | CN 101400362 A | 01-04-2009 |
| | | EP 1991247 A1 | 19-11-2008 |
| | | JP 5441416 B2 | 12-03-2014 |
| | | JP 2009526864 A | 23-07-2009 |
| | | JP 2013018783 A | 31-01-2013 |
| | | US 2009312363 A1 | 17-12-2009 |
| | | US 2013018103 A1 | 17-01-2013 |
| | | WO 2008091349 A1 | 31-07-2008 |
| US 2013065963 A1 | 14-03-2013 | CA 2800212 A1 | 27-01-2005 |
| | | EP 2258677 A2 | 08-12-2010 |
| | | US 2005014839 A1 | 20-01-2005 |
| | | US 2005032831 A1 | 10-02-2005 |
| | | US 2009163543 A1 | 25-06-2009 |
| | | US 2011098504 A1 | 28-04-2011 |
| | | US 2013065963 A1 | 14-03-2013 |

EPO FORM P0459

For more details about this annex : see Official Journal of the European Patent Office, No. 12/82